# EUROPÄISCHE PATENTSCHRIFT

(11) **EP 3 145 455 B1**
(45) Veröffentlichungstag und Bekanntmachung des Hinweises auf die Patenterteilung: **09.05.2018**
(21) Anmeldenummer: 15727912.6
(22) Anmeldetag: 22.05.2015
(51) Int. Cl.: A61F 5/01

(54) **ORTHOPÄDISCHE VORRICHTUNG ZUR BEGRENZUNG DER BEWEGUNG EINES ZWISCHEN EINEM ERSTEN UND ZWEITEN KÖRPERBEREICH ANGEORDNETEN GELENKS**
ORTHOPEDIC DEVICE FOR LIMITING THE MOVEMENT OF A JOINT ARRANGED BETWEEN A FIRST AND A SECOND BODY REGION
DISPOSITIF ORTHOPÉDIQUE PERMETTANT DE LIMITER LE MOUVEMENT D'UNE ARTICULATION SE TROUVANT ENTRE DEUX PARTIES DU CORPS

(30) Priorität: 23.05.2014 DE 102014107335
(43) Veröffentlichungstag der Anmeldung: 29.03.2017
(73) Patentinhaber: Betterguards Technology GmbH, 10625 Berlin (DE)
(72) Erfinder: BICHLER, Vinzenz, 10623 Berlin (DE)
(74) Vertreter: Okoampah, Rene
(86) Internationale Anmeldenummer: PCT/EP2015/061450
(87) Internationale Veröffentlichungsnummer: WO 2015/177357

(56) Entgegenhaltungen:
- DE-A1-102012 011 433
- US-A- 4 471 538
- US-A- 5 712 011
- US-A1- 2007 010 772
- US-A1- 2013 296 755
- US-A1- 2014 015 176

## Beschreibung

### Technisches Gebiet

Die vorliegende Erfindung betrifft eine orthopädische Vorrichtung zur Begrenzung der Bewegung eines zwischen einem ersten und einem zweiten Körperbereich angeordneten Gelenks, umfassend zumindest eine an dem ersten Körperbereich fixierbare Aufnahme und einen an dem zweiten Körperbereich fixierbaren und relativ zu der Aufnahme bewegbaren Auszugskörper, wobei senkrecht zu einer Auszugsrichtung zwischen der Aufnahme und dem Auszugskörper ein mit einem dilatanten Fluid gefüllter Durchgangsquerschnitt bereitgestellt ist.

### Stand der Technik

Schädigungen im Bereich der am Bewegungszyklus beteiligten Gelenke zählen zu einer der wichtigsten Unfallschwerpunkte. Dabei sind besonders die so genannten "Stolper-, Rutsch- und Sturzunfälle" (SRS-Unfälle) hervorzuheben, welche als Verletzungsursache bei Wege-, Arbeits- und Sportunfällen am häufigsten vorkommen. Diese Unfälle können einzeln oder in Kombination zu erheblichen Verletzungen führen. Überwiegend betroffen sind vor allem größere Gelenke, wie das obere und untere Sprunggelenk (articulatio talocuralis und articulatio talotarsalis), das Handgelenk (articulatio manus) sowie das Kniegelenk (articulatio genus). Dabei ist eine Verletzungstendenz in diesem Bereich auch sportmedizinisch zu verzeichnen.

Durch die SRS-Unfälle kommt es häufig zu einer gewaltsamen Überdehnung des Halteapparats des Gelenks (Gelenkkapsel, Bänder, Sehnen, Knochen) über den physiologisch maximal möglichen Winkel. Diese Überdehnung ist auch bekannt unter den Begriffen Distorsion beziehungsweise Verstauchung. Da dies beim Sprung- und Handgelenk oft in Supinationsrichtung auftritt, spricht man in diesem Fall von einem so genannten Supinationstrauma. Dabei handelt es sich um ein Synonym für eine Verletzung, bei der eine über den physiologischen Bewegungsspielraum erfolgte Supination des Fußes zusammen mit einer Plantarflexion zu einer Schädigung der lateralen Knochenstrukturen und des lateralen Kolateralbandapparates führt. Supinationstraumata äußern sich zunächst in starken Schmerzen, die meist mit einer Schwellung der Gliedmaße und der Einwanderung eines Hämatoms (im Fall von ruptierten Bändern) verbunden sind. Werden solche Begleiterscheinungen nicht erkannt und medizinisch ausreichend versorgt, können Spätfolgen, wie beispielsweise eine chronische Instabilität des Gelenks oder eine Gelenkarthrose, eintreten. Die Verletzungsursachen für Supinationstraumata sind meist hohe Supinations- (Inversions-) Geschwindigkeit, sowie ein Überschreiten eines für die Verletzung verantwortlichen kritischen Winkels, bspw. beim Umknicken am Sprunggelenk. So kann bspw. eine kritische Winkelgeschwindigkeit beim Umknicken am Sprunggelenk von über ca. 300/s zu einer Verletzung führen. Im Folgenden werden Kennwerte unterhalb dieser kritischen Winkelgeschwindigkeit bei Ausführungsvarianten am Sprunggelenk als physiologische Bewegungen bezeichnet. Oberhalb dieses kritischen Kennwertes werden Bewegungen als unphysiologische Bewegungen bezeichnet. Die auftretenden Geschwindigkeiten sind auf Kräfte beziehungsweise Beschleunigungen zurückzuführen, welche auf die Körperteile und Gelenke wirken.

Ein sehr wichtiger Faktor für den positiven Heilungsverlauf eines Supinationstraumas ist die Stabilisierung der Gelenke des Patienten, zur Verletzungsprävention oder während der prä- oder postoperativen Versorgung. Dabei wird die Stabilisierung der Gelenke in der Regel über Tapeverbände, Funktionssicherungsorthesen, Stabilisierungsorthesen, Bandagen und/oder stabile Schuhe mit variabler Schafthöhe und optionalen Verstärkungselementen erreicht.

Unter Orthese wird im Allgemeinen und in dieser Anmeldung ein funktionssicherndes, körperumschließendes oder körperanliegendes Hilfsmittel verstanden, das von seiner physikalischen/mechanischen Leistung konstruktiv stabilisiert, immobilisiert, mobilisiert, entlastet, korrigiert, retiniert, fixiert, redressiert und/oder ausgefallene Körperfunktionen ersetzt.

Dabei kommen insbesondere dynamischen Orthesen zur Regulierung der Kraft zwischen relativ zueinander bewegbaren Körperteilen zum Einsatz. Unter anderem sind Orthesen bekannt, deren Beweglichkeit von dem Scherverhalten eines dilatanten Fluids abhängt.

Für eine adaptive Bewegungsbegrenzung kommen dilatante, scherverdickende Materialien, wie beispielsweise Copolymerdispersionen oder -fluide, welche bei dem Auftreten von hohen Scherkräften und einer damit verbundenen hohen Schwergeschwindigkeit eine höhere Viskosität aufweisen, zum Einsatz. Je größer die aufgebrachte Scherung ist, desto viskoser beziehungsweise zähflüssiger verhält sich das Fluid. Entsprechend werden dilatanten Fluiden auch scherverfestigende beziehungsweise scherverdickende Eigenschaften zugeschrieben.

Orthesen, welche sich die Eigenschaften solcher dilatanten Fluide zu Eigen machen, können beispielsweise eine Aufnahme und einen Auszugskörper aufweisen. Dabei ist die Aufnahme mit dem dilatanten Fluid gefüllt. Der Auszugskörper ragt in die Aufnahme hinein, sodass zumindest der mit der Aufnahme überlappende Bereich des Auszugskörpers von dem dilatanten Fluid umgeben ist. Der Auszugskörper kann dabei an einem ersten Körperteil angebracht sein, die Aufnahme hingegen an einem sich zu dem ersten Körperteil relativ bewegbaren zweiten Körperteil.

Bei einer geringen Kräfteeinwirkung kann der Auszugskörper in dem dilatanten Fluid nahezu uneingeschränkt bewegt werden. Bei hoher Krafteinwirkung und einem daraus resultierenden starken beziehungsweise abrupten Zug am Auszugskörper kommt es zu einem Überschreiten der kritischen Schergeschwindigkeit in dem dilatanten Fluid. Dies hat je nach verwendetem Fluid einen Anstieg der Zugspannung um den Faktor 100 bis zum Faktor 1000 zur Folge. Der Anstieg der Zugspannung hat eine vernetzende Wirkung auf das dilatante Fluid, wodurch der eine hohe Krafteinwirkung erfahrende Auszugskörper durch den starken Anstieg der Viskosität des dilatanten Fluids im selbigen gehalten wird und so keine oder nur eine verminderte Bewegung erfährt.

Die US 5,712,011 zeigt beispielsweise eine zerrresistente Einheit, wobei ein erster Teil einen nichtdehnbaren Abschnitt mit einer Oberfläche aufweist, welche mit der Oberfläche eines zweiten, nichtdehnbaren Teils überlappt. Die beiden Teile sind dabei derart angeordnet, dass sie eine translatorische, im Wesentlichen parallele Bewegung relativ zueinander zulassen. Zwischen den Teilen ist eine scherverfestigende Zusammensetzung angeordnet, welche einen variablen Widerstand gegen die translatorische Bewegung des zweiten Teils relativ zu dem ersten Teil aufbringt. Dabei resultiert der Widerstand aus den auf die scherverfestigende Zusammensetzung wirkenden Scherkräfte, welche sich aus der Relativbewegung der nichtdehnbaren Teile zueinander ergeben. Die beiden überlappenden Teile sowie die scherverfestigende Zusammensetzung sind dabei von einer Hülle umgeben.

DE 10 2012 011433 A1 offenbart eine Orthopädische Vorrichtung zur Begrenzung der Bewegung eines zwischen einem ersten und einem zweiten Körperbereich angeordneten Gelenks, umfassend zumindest eine an dem ersten Körperbereich fixierbare Aufnahme und einen an dem zweiten Körperbereich fixierbaren und relativ zu der Aufnahme bewegbaren Auszugskörper, wobei senkrecht zu einer Auszugsrichtung zwischen der Aufnahme und dem Auszugskörper ein mit einem dilatanten Fluid gefüllter Durchgangsquerschnitt bereitgestellt ist. Bestehende Vorrichtungen ermöglichen bisher entweder eine starke Schutzwirkung mit damit einhergehender Immobilisierung, oder aber eine leichte Schutzwirkung, die bei einem Verletzungsfall nur unzureichend schützt. Für verschiedene Lastszenarien ist somit ein Wechsel der Vorrichtung beziehungsweise eine manuelle Voreinstellung nötig, um die Eigenschaften der Vorrichtung dem Heilungsverlauf anzupassen.

Eine Aufgabe der vorliegenden Erfindung ist es, eine verbesserte orthopädische Vorrichtung bereitzustellen.

### Darstellung der Erfindung

Ausgehend von dem bekannten Stand der Technik ist es eine Aufgabe der vorliegenden Erfindung, eine orthopädische Vorrichtung zur Begrenzung der Bewegung eines zwischen einem ersten und einem zweiten Körperbereich angeordneten Gelenks bereitzustellen, welche die Anwendung der orthopädischen Vorrichtung erleichtert und den Einsatzbereich vergrößert.

Diese Aufgabe wird mittels einer Vorrichtung mit den Merkmalen des Anspruchs 1 gelöst. Vorteilhafte Ausgestaltungen ergeben sich aus den Unteransprüchen.

Entsprechend wird eine orthopädische Vorrichtung zur Begrenzung der Bewegung eines zwischen einem ersten und einem zweiten Körperbereich angeordneten Gelenks, umfassend zumindest eine an dem ersten Körperbereich fixierbare Aufnahme und einen an dem zweiten Körperbereich fixierbaren und relativ zu der Aufnahme bewegbaren Auszugskörper, wobei senkrecht zu einer Auszugsrichtung zwischen der Aufnahme und dem Auszugskörper ein mit einem dilatanten Fluid gefüllter Durchgangsquerschnitt bereitgestellt ist, angegeben. Erfindungsgemäß ist dabei mindestens ein Durchgangsbegrenzungselement zur Veränderung des Durchgangsquerschnitts vorgesehen.

Mindestens ein Durchgangsbegrenzungselement kann bedeuten, dass genau ein Durchgangsbegrenzungselement in der orthopädischen Vorrichtung bereitgestellt ist. Alternativ kann mindestens ein Durchgangsbegrenzungselement auch bedeuten, dass mehrere Durchgangsbegrenzungselemente in der orthopädischen Vorrichtung bereitgestellt sind, wie beispielsweise zwei, drei, vier, fünf, zehn, 20, 40, 50, 64 oder 100 Durchgangsbegrenzungselemente.

Der Durchgangsquerschnitt stellt den auf einer Ebene senkrecht zur Auszugsrichtung, über die Länge des Auszugskörpers gesehen, kleinsten Abstand zwischen dem Auszugskörper und der Aufnahme dar. Somit bildet der Durchgangsquerschnitt den über die Länge des Auszugskörpers gesehenen kleinsten Durchgang für ein dilatantes Fluid zwischen dem Auszugskörper und der Aufnahme.

Das mindestens eine Durchgangsbegrenzungselement kann am Auszugskörper und/oder an der Aufnahme angeordnet sein. Alternativ können auch mehrere Durchgangsbegrenzungselemente an dem Auszugskörper und/oder der Aufnahme angeordnet sein. Das mindestens eine Durchgangsbegrenzungselement ist dazu vorgesehen, den Durchgangsquerschnitt zu verändern. In einer Ausgangsposition des mindestens einen Durchgangsbegrenzungselements ist der größtmögliche Durchgangsquerschnitt zwischen dem Auszugskörper und der Aufnahme bereitgestellt. Abhängig von der Geschwindigkeit, mit welcher sich der Auszugskörper relativ zu der Aufnahme bewegt, insbesondere aus ihr herausgezogen wird, kann das mindestens eine Durchgangsbegrenzungselement eine von der Ausgangsposition abweichende Position einnehmen, durch welche der Durchgangsquerschnitt zwischen dem Auszugskörper und der Aufnahme reduziert wird.

In einer maximalen Begrenzungsposition ist die größtmögliche Reduzierung des Durchgangsquerschnitts durch das mindestens eine Durchgangsbegrenzungselement bereitgestellt.

Dadurch, dass der Durchgangsquerschnitt mit Hilfe des mindestens einen Durchgangsbegrenzungselements verändert werden kann, können die Viskosität und die Scherrate des dilatanten Fluids in der orthopädischen Vorrichtung beeinflusst werden. So kann eine Reduktion des Durchgangsquerschnitts dazu führen, dass das dilatante Fluid bei gleicher Scherrate eine deutlich höhere Viskosität aufweist. Darüber hinaus kann aufgrund eines reduzierten Durchgangsquerschnitts ein Dilatanzsprung, d.h. das Einsetzen der Scherverfestigung schon bei deutlich geringeren Scherraten erzielt werden.

Die Position des mindestens einen Durchgangsbegrenzungselements, und damit die Größe des Durchgangsquerschnitts, liegt dabei in einem Abhängigkeitsverhältnis mit der Relativgeschwindigkeit des Auszugskörpers gegenüber der Aufnahme. Bei geringen Geschwindigkeiten, welche für das mit der orthopädischen Vorrichtung zu schützende Gelenk unkritisch sind, sogenannte physiologische Geschwindigkeiten, bleibt das mindestens eine Durchgangsbegrenzungselement in der Ausgangsposition bzw. weicht nur geringfügig von dieser ab, so dass der Durchgangsquerschnitt zwischen dem Auszugskörper und der Aufnahme nur geringfügig reduziert wird. Somit können langsame Relativbewegungen des Auszugskörpers gegenüber der Aufnahme aufgrund einer geringen Viskosität des dilatanten Fluids ermöglicht werden.

Mit zunehmender Relativgeschwindigkeit, insbesondere Auszugsgeschwindigkeit, des Auszugskörpers gegenüber der Aufnahme, wird der Durchgangsquerschnitt zwischen dem Auszugskörper und der Aufnahme zunehmend durch das mindestens eine Durchgangsbegrenzungselement reduziert. Durch die Verringerung des Durchgangsquerschnitts zwischen dem Auszugskörper und der Aufnahme steigt die Viskosität in dem dilatanten Fluid. Darüber hinaus reduziert sich die nötige Scherrate zum Einsatz der Scherverfestigung.

Mit einer zunehmenden Auslenkung des mindestens einen Durchgangsbegrenzungselements steigt auch der durch das mindestens eine Durchgangsbegrenzungselement hervorgerufene Strömungswiderstand im dilatanten Fluid. Aufgrund der zunehmenden Auslenkung vergrößert sich eine projizierte Fläche in Auszugsrichtung des mindestens einen Durchgangsbegrenzungselements, was einen vergrößerten Auszugswiderstand unter einer vermehrten Bildung von Turbulenzen und Wirbeln zur Folge hat. Die projizierte Fläche entsteht, wenn man das mindestens eine Durchgangselement parallel zur Auszugsrichtung auf eine Fläche senkrecht zur Auszugsrichtung projiziert. Durch die entstehenden Turbulenzen und Wirbel kommt es im dilatanten Fluid zu einer zusätzlichen, von der Relativgeschwindigkeit abhängigen Verfestigung. Diesen Effekt der Dilatanz wird als pseudodilatantes Verhalten bezeichnet.

Somit kann erreicht werden, dass die Relativbewegung des Auszugskörpers gegenüber der Aufnahme eingeschränkt bis hin zu vollständig unterdrückt wird.

In einer bevorzugten Ausführungsform ist das mindestens eine Durchgangsbegrenzungselement plattenförmig, bevorzugt scheibenförmig, besonders bevorzugt schuppenförmig, und/oder stabförmig, bevorzugt borstenförmig.

Dadurch kann der Durchgangsquerschnitt in einer von der Ausgangsposition abweichenden Position des mindestens einen Durchgangsbegrenzungselements durch die Oberflächen des mindestens einen Durchgangsbegrenzungselements reduziert werden. Je größer der Bereich des Durchgangsquerschnitts, welcher von der Oberfläche des mindestens einen Durchgangsbegrenzungselements abgedeckt wird, desto kleiner ist der Durchgangsquerschnitt.

Das mindestens eine plattenförmige und/oder stabförmige Durchgangsbegrenzungselement zum Auslenken aus einer Ausgangsposition kann dabei in der Ausgangsposition parallel zur Längsrichtung des Auszugskörpers und der Aufnahme angeordnet sein. Gegenüber dem ersten Ende des mindestens einen Durchgangsbegrenzungselements, welches fest mit dem Auszugskörper oder der Aufnahme verbunden ist, ist ein zweites Ende des mindestens einen Durchgangsbegrenzungselements bereitgestellt. Dabei weist das mindestens eine Durchgangsbegrenzungselement eine erste Oberfläche, welche in der Ausgangsposition dem Auszugskörper gegenüberliegt und eine zweite Oberfläche, welche in der Ausgangsposition der Aufnahme gegenüberliegt, auf. Das zweite freie Ende des mindestens einen plattenförmigen und/oder stabförmigen Durchgangsbegrenzungselements zeigt in der Ausgangsposition in die Auszugsrichtung.

Das plattenförmige bevorzugt scheibenförmige, besonders bevorzugt schuppenförmige, und/oder stabförmige, bevorzugt borstenförmige mindestens eine Durchgangsbegrenzungselement zum Auslenken aus einer Ausgangsposition kann somit durch die Auslenkung aus der Ausgangsposition, welche es in Abhängigkeit der Auszugsgeschwindigkeit erfährt, den Durchgangsquerschnitt verändern, so dass eine Beeinflussung der Viskosität des dilatanten Fluids in Abhängigkeit von der Auszugsgeschwindigkeit ermöglicht wird. Insbesondere sorgt das mindestens eine Durchgangsbegrenzungselement für eine Zunahme von Turbulenzen und Wirbeln im dilatanten Fluid.

In einer weiter bevorzugten Ausführungsform ist das mindestens eine Durchgangsbegrenzungselement zum Auslenken aus einer Ausgangsposition elastisch und an einem ersten Ende fest, bevorzugt drehfest, mit dem Auszugskörper oder der Aufnahme verbunden.

Beim Herausziehen des Auszugskörpers aus der Aufnahme wird das mindestens eine Durchgangsbegrenzungselement mit dilatantem Fluid umströmt. Aufgrund der elastischen Eigenschaften des mindestens einen Durchgangsbegrenzungselements und der Auswirkung des umströmenden Fluids, kann das mindestens eine Durchgangsbegrenzungselement in Abhängigkeit von der Auszugsgeschwindigkeit von der Ausgangsposition bis hin zu der maximalen Begrenzungsposition gebogen werden.

Dadurch kann der Durchgangsquerschnitt, welcher von dem Abstand des zweiten Endes des mindestens einen Durchgangsbegrenzungselements und der Aufnahme und/oder des Auszugskörpers abhängt, reduziert werden.

Das mindestens eine elastische Durchgangsbegrenzungselement kann somit durch die Auslenkung aus der Ausgangsposition, welche es in Abhängigkeit der Auszugsgeschwindigkeit erfährt, den Durchgangsquerschnitt verändern, so dass eine Beeinflussung der Viskosität des dilatanten Fluids in Abhängigkeit von der Auszugsgeschwindigkeit ermöglicht wird. Insbesondere sorgt das mindestens eine Durchgangsbegrenzungselement für eine Zunahme von Turbulenzen und Wirbeln im dilatanten Fluid.

Wird der Auszugskörper entgegen der Auszugsrichtung in die Aufnahme hinein bewegt, unterstützt das das mindestens eine Durchgangsbegrenzungselement umströmende dilatanten Fluid die Rückkehr des mindestens eine Durchgangsbegrenzungselements in die Ausgangsposition.

Darüber hinaus kann das mindestens eine Durchgangsbegrenzungselement aufgrund seiner elastischen Eigenschaften bei Reduktion oder Wegfall der Auszugsgeschwindigkeit wieder in die Ausgangsposition zurückkehren.

In einer bevorzugten Weiterbildung ist das mindestens eine Durchgangsbegrenzungselement zum Auslenken aus einer Ausgangsposition an einem ersten Ende über ein Gelenk, bevorzugt ein Scharnier, besonders bevorzugt ein Filmscharnier, mit dem Auszugskörper oder der Aufnahme verbunden.

Das mindestens eine Durchgangsbegrenzungselement kann somit in einer Ausgangsposition eingeklappt sein, so dass es an dem Auszugskörper und/oder der Aufnahme anliegt, wobei der größtmögliche Durchgangsquerschnitt zwischen der Aufnahme und/oder dem Auszugskörper und dem mindestens einen Durchgangsbegrenzungselement, insbesondere einem zweiten Ende des mindestens einen Durchgangsbegrenzungselements, bereitgestellt ist.

Das zweite Ende zeigt in der Ausgangsposition bevorzugt in Auszugsrichtung. Wird der Auszugskörper aus der Aufnahme herausgezogen, klappt das mindestens eine Durchgangsbegrenzungselement aufgrund einer Interaktion mit dem umströmenden dilatanten Fluid heraus. Dabei nähert sich das zweite Ende des mindestens einen Durchgangsbegrenzungselements der Aufnahme und/oder dem Auszugskörper an, wodurch der Durchgangsquerschnitt reduziert wird.

Das Ausklappen des mindestens einen Durchgangsbegrenzungselements wird durch das Gelenk ermöglicht, welches das mindestens eine Durchgangsbegrenzungselement an dem ersten Ende mit dem Auszugskörper oder der Aufnahme verbindet.

Bevorzugt kann das mindestens eine Durchgangsbegrenzungselement bis zu einer maximalen Begrenzungsposition ausgeklappt werden. Die Begrenzungsposition kann beispielsweise durch einen Anschlag am ersten Ende des mindestens einen Durchgangsbegrenzungselements, welcher mit dem Gelenk oder der Oberfläche des Auszugskörpers oder der Aufnahme anschlägt, bereitgestellt werden.

Wird der Auszugskörper in die Aufnahme hineingedrückt, umströmt das dilatante Fluid das mindestens eine Durchgangsbegrenzungselement derart, dass es hin zu seiner Ausgangsposition geklappt wird. Insbesondere drückt das dilatante Fluid auf eine zweite Oberfläche des mindestens einen Durchgangsbegrenzungselements und sorgt so für die Rückstellung des mindestens einen Durchgangsbegrenzungselements in seine Ausgangsposition.

Zusätzlich kann das Gelenk eine Rückstellfeder zum Zurückstellen des mindestens einen Durchgangsbegrenzungselements in seine Ausgangsposition nach dem Wegfall der Auszugsbewegung aufweisen.

Das Gelenk kann vorteilhafterweise in Form eines Scharniers ausgestaltet sein. Dabei kann das Scharnier an dem ersten Ende des mindestens einen Durchgangsbegrenzungselements an dem Auszugskörper oder der Aufnahme angebracht sein, um somit das Aus- und Einklappen des mindestens einen Durchgangsbegrenzungselements zu ermöglichen.

Darüber hinaus kann das Scharnier in Form eines Filmscharniers ausgebildet sein. Dadurch lässt sich das mindestens eine Durchgangsbegrenzungselement mit dem Auszugskörper oder der Aufnahme einteilig ausbilden. Das Filmscharnier kann aus einer dünnwandigen Verbindung bestehen, welche durch ihre Biegsamkeit eine begrenzte Drehbewegung der verbundenen Teile ermöglicht. Die einteilige Ausführung kann beispielsweise aus einem Mehrkomponenten Spritzguss bestehen.

Die Verbindung des mindestens einen Durchgangsbegrenzungselements mit dem Auszugskörper oder der Aufnahme über ein Gelenk, Scharnier oder Filmscharnier, ermöglicht somit, dass das mindestens eine Durchgangsbegrenzungselement aus- und eingeklappt werden kann. Dadurch ist es möglich, den Durchgangsquerschnitt zwischen dem Auszugskörper oder der Aufnahme und dem mindestens einen Durchgangsbegrenzungselement zu verändern.

In einer weiter bevorzugten Ausführungsform ist das mindestens eine Durchgangsbegrenzungselement in einer Ausgangsposition zu dem Auszugskörper in einem spitzen Winkel angeordnet.

Das mindestens eine Durchgangsbegrenzungselement kann dabei wie ein Anker wirken. So ist ein erstes Ende des mindestens einen Durchgangsbegrenzungselements fest oder über ein Gelenk mit dem Auszugskörper verbunden. Das zweite Ende des mindestens einen Durchgangsbegrenzungselements ragt dabei wie die Spitze der Flunke eines Ankers von dem Auszugskörper hervor. Dabei zeigt das zweite Ende des mindestens einen Durchgangsbegrenzungselements in der Ausgangsposition im Wesentlichen in die Auszugsrichtung.

Wird der Auszugskörper in Auszugsrichtung bewegt, strömt das dilatante Fluid auf eine erste, in Bezug auf den Auszugskörper innen liegende Oberfläche und übt auf diese eine Kraft aus. Je nach Befestigung des mindestens einen Durchgangsbegrenzungselements an dem Auszugskörper klappt oder biegt sich das mindestens eine Durchgangsbegrenzungselement hin zu der Aufnahme und verringert dadurch den Durchgangsquerschnitt zwischen der Aufnahme und dem zweiten Ende des mindestens einen Durchgangsbegrenzungselements.

Dabei ist es besonders vorteilhaft, wenn einem Durchgangsbegrenzungselement auf der anderen Seite des Auszugskörpers ein zweites Durchgangsbegrenzungselement gegenüberliegt. Dadurch kann sichergestellt werden, dass der Auszugskörper parallel zu der Aufnahme aus dieser gezogen wird. Somit können Kraftmomente, welche aufgrund der Hebelwirkung ausgehend von dem auf die Durchgangsbegrenzungselemente wirkenden Fluidkräfte entstehen können, ausgeglichen werden, so dass der Auszugskörper in einer Ausrichtung parallel zur Aufnahme gehalten wird und nicht an die innere Oberfläche der Aufnahme gedrückt wird.

Insbesondere kann so ein gleichbleibender Abstand zwischen den sich gegenüberliegenden Durchgangsbegrenzungselementen und der Aufnahme bereitgestellt werden.

In einer weiter bevorzugten Ausgestaltung ist der Auszugskörper von mindestens einem Durchgangsbegrenzungselement schirmförmig umgeben.

Dabei kann der Auszugskörper von mindestens einem durchgehenden Durchgangsbegrenzungselement umgeben sein, welches den Auszugskörper in Bezug auf die Auszugsrichtung umfänglich umläuft.

In einer Ausgangsposition bilden das mindestens eine schirmförmige Durchgangsbegrenzungselement und der Auszugskörper einen geschlossenen Schirm. Dabei bildet ein erster, den Auszugskörper umlaufender Rand eine drehfeste Verbindung oder Gelenkverbindung mit dem mindestens einen Durchgangsbegrenzungselement. Ein zweiter, den Auszugskörper umlaufender freier Rand des Schirms kann sich durch Herausziehen des Auszugskörpers aus der Aufnahme von dem Auszugskörper entfernen und somit das schirmförmige Begrenzungselement aufspannen. Dadurch kann der Durchgangsquerschnitt zwischen der Aufnahme und dem zweiten umlaufenden Rand des mindestens einen schirmförmigen Durchgangsbegrenzungselements reduziert werden.

Das mindestens eine schirmförmige Durchgangsbegrenzungselement kann bei einer einteiligen Ausgestaltung ein elastisches Material, bevorzugt Gummi oder Kautschuk aufweisen.

Alternativ können auch mehrere einzelne Durchgangsbegrenzungselemente um den Auszugskörper herum angeordnet sein und so einen mehrteiligen Schirm um den Auszugskörper herum bilden. Dabei können die einzelnen Durchgangsbegrenzungselemente insbesondere fächerartig, d.h., teilweise überlappend um den Auszugskörper herum angeordnet sein. Auf diese Weise bestehen geringere Anforderungen an die Elastizität der Durchgangsbegrenzungselemente bei der Reduktion des Durchgangsquerschnitts. So kann eine Vergrößerung des Umfangs des zweiten umlaufenden Randes, welcher aus dem zweiten Ende der Durchgangsbegrenzungselemente gebildet wird, durch eine Reduktion der überlappenden Bereiche der Durchgangsbegrenzungselemente untereinander, welche mit einer Bewegung des zweiten Endes hin zu der Aufnahme einhergeht, erreicht werden.

Die schirmförmige Ausgestaltung des mindestens einen Durchgangsbegrenzungselements stellt neben der Möglichkeit der Erhöhung der Viskosität aufgrund der Reduktion des Durchgangsquerschnitts zusätzlich einen erhöhten Strömungswiderstand bereit. Somit wird der Auszugskörper beim Herausziehen aus der Aufnahme zusätzlich aufgrund der schirmförmigen Ausgestaltung abgebremst bzw. gestoppt.

In einer bevorzugten Ausgestaltung weist der Auszugskörper und/oder die Aufnahme mindestens eine Aussparung auf, in welche mindestens ein Durchgangsbegrenzungselement in einer Ausgangsposition versenkt ist.

Dadurch kann der gesamte Abstand des Auszugskörpers zur Aufnahme senkrecht zur Auszugsrichtung als Durchgangsquerschnitt für das dilatante Fluid genutzt werden. Durch die für das zu schonende Gelenk unkritischen Krafteinwirkungen auf die orthopädische Vorrichtung und die damit einhergehenden physiologischen Geschwindigkeiten können Auszugskörper und Aufnahme relativ zueinander bewegt werden, wobei der Durchgangsquerschnitt nicht von dem mindestens einen Durchgangsbegrenzungselement beeinflusst beziehungsweise reduziert wird.

Insgesamt lassen sich dadurch geringe Bauteilhöhen der orthopädischen Vorrichtung realisieren. So bedarf es keines zusätzlichen Abstands zwischen Auszugskörper und Aufnahme für die Bereitstellung des mindestens einen Durchgangsbegrenzungselements.

Ein weiterer Vorteil liegt darin, dass durch eine versenkte Anordnung der Durchgangsbegrenzungselemente in der Ausgangsposition keine Vorsprünge von dem Auszugskörper oder der Aufnahme aufgrund des mindestens einen Durchgangsbegrenzungselements bereitgestellt werden, wodurch weniger viskositätserhöhende Wirbel und Turbulenzen in dem dilatanten Fluid hervorgerufen werden, bevor eine Erhöhung der Viskosität des dilatanten Fluids gewünscht ist. Dadurch kann insbesondere bei einer physiologischen Bewegung ohne erhöhten Kraftaufwand einer verfrühten Bewegungsbegrenzung der orthopädischen Vorrichtung entgegengewirkt werden.

Bei hoher Krafteinwirkung hingegen kann das Umströmen der ersten und zweiten Oberfläche des mindestens einen Durchgangsbegrenzungselements dieses aus seiner Ausgangsposition auslenken und somit das zweite Ende des mindestens einen Durchgangsbegrenzungselements hin zu der Aufnahme oder dem Auszugskörper bewegen und so den Durchgangsquerschnitt zwischen dem zweiten Ende des mindestens einen Durchgangsbegrenzungselements und der Aufnahme reduzieren.

Um ein Auslenken des mindestens einen Durchgangsbegrenzungselements zu begünstigen, kann das mindestens eine Durchgangsbegrenzungselement in der Ausgangsposition gegenüber der Auszugsrichtung geringfügig schräg angeordnet sein.

Bevorzugt liegt einem in einer Aussparung in dem Auszugskörper angeordneten mindestens einen Durchgangsbegrenzungselement auf der anderen Seite des Auszugskörpers senkrecht zur Auszugsrichtung ein weiteres, in einer weiteren Auslenkung des Auszugskörpers angeordnetes Durchgangsbegrenzungselement gegenüber. Bevorzugt weist der Auszugskörper zu jedem Durchgangsbegrenzungselement ein weiteres Durchgangsbegrenzungselement, welches horizontal in entgegengesetzte Richtung bezüglich dem ersten Durchgangsbegrenzungselement bewegbar ist. Die Möglichkeit den Durchgangsquerschnitt durch gegenüberliegende Durchgangsbegrenzungselemente zu reduzieren, hat den Vorteil, dass die auf den Auszugskörper wirkenden Kräfte ausgehend von den Durchgangsbegrenzungselementen ausgeglichen werden können. Somit kann der Auszugskörper trotz Interaktion der Durchgangsbegrenzungselemente mit dem dilatanten Fluid parallel zur Aufnahme bewegt werden ohne gegen die innere Oberfläche der Aufnahme gedrückt zu werden.

In einer weiteren Ausführungsform weist der Auszugskörper mindestens einen Durchgang zum Bereitstellen eines zusätzlichen Fließweges für das dilatante Fluid auf, in welcher mindestens ein Durchgangsbegrenzungselement in einer Ausgangsposition versenkt ist.

Dadurch kann in einer von der Ausgangsposition abweichenden Position des mindestens einen Durchgangsbegrenzungselements das dilatante Fluid zusätzlich durch den Durchgang des Auszugskörpers fließen.

Beginnt sich das mindestens eine Durchgangsbegrenzungselement aus seiner Ausgangsposition auszulenken, wird zunächst der Durchgangsquerschnitt zwischen Auszugskörper und Aufnahme reduziert. Bewegt sich das zweite Ende des mindestens einen Durchgangsbegrenzungselements weiter hin zu der Aufnahme, entsteht zwischen dem zweiten Ende und dem Auszugskörper ein weiterer Fließweg für das dilatante Fluid, welcher durch den Durchgang des Auszugskörpers hindurch führt. Auf diese Weise können zwei Fließwege für das dilatante Fluid bereitgestellt werden, welche jeweils einen kleineren Durchgangsquerschnitt aufweisen, als der Durchgangsquerschnitt, welcher bereitgestellt wird, wenn sich das mindestens eine Durchgangsbegrenzungselement in der Ausgangsposition befindet.

Im Vergleich zu der Viskosität des dilatanten Fluids in der Ausgangsposition des mindestens einen Durchgangsbegrenzungselements herrscht in den beiden Fließwegen aufgrund des jeweils kleineren Durchgangsquerschnitts eine höhere Viskosität.

Weist der Auszugskörper mehrere Durchgangsbegrenzungselemente auf, so sollten diese in ihren Ausgangspositionen derart angeordnet sein, dass sie senkrecht zur Auszugsrichtung in entgegengesetzte Richtungen zueinander ausgelenkt werden. Beispielsweise können die Durchgangsbegrenzungselemente unterschiedlich schräggestellte Ausgangspositionen aufweisen, welche eine entgegengesetzte Auslenkung begünstigen. Alternativ können für den Fall, dass die Durchgangsbegrenzungsmittel über Gelenke mit dem Auszugskörper verbunden sind, die Gelenke derart ausgebildet sein, dass sie sich, bezogen auf zwei Durchgangsbegrenzungselemente, nur in entgegengesetzte Richtungen auslenken lassen.

In einer weiter bevorzugten Ausgestaltung weist das mindestens eine Durchgangsbegrenzungselement mindestens einen Vorsprung auf, welcher in den Durchgangsquerschnitt hineinragt.

Wird der Auszugskörper mit hoher Geschwindigkeit aus der Aufnahme herausgezogen, drückt das dilatante Fluid auf den mindestens einen Vorsprung, wodurch dieser aufgrund der entstehenden Hebelwirkung das mindestens eine Durchgangsbegrenzungselement aus seiner Ausgangsposition lenkt.

Der mindestens eine Vorsprung ist dabei derart ausgelegt, dass er bei geringen Auszugsgeschwindigkeiten, welche durch physiologische Bewegungen hervorgerufen werden, in dem dilatanten Fluid lediglich vernachlässigbar geringe Verwirbelungen erzeugt und ein Auslenken des mindestens einen Durchgangsbegrenzungselements aus seiner Ausgangsposition nicht erfolgt.

In einer weiter bevorzugten Ausführungsform weist das mindestens eine Durchgangsbegrenzungselement eine Tasche auf, um einen Teil des dilatanten Fluids in einen Durchgang des Auszugskörpers umzulenken, wobei eine Taschenöffnung in die Bewegungsrichtung des Auszugskörpers, bevorzugt die Auszugsrichtung des Auszugskörpers zeigt.

Dadurch kann das dilatante Fluid bei geringen Auszugsgeschwindigkeiten, welche beispielsweise auf physiologische Körperbewegungen zurückzuführen sind, zum einen durch den Durchgangsquerschnitt zwischen Durchgangsbegrenzungselement und Aufnahme fließen, zum anderen kann das dilatante Fluid durch den zusätzlichen Durchgangsquerschnitt der Tasche durch den Auszugskörper hindurchfließen. Die Viskosität des dilatanten Fluids in den beiden Fließwegen wird jeweils von der Größe des Durchgangsquerschnitts bestimmt, durch welchen das dilatante Fluid hindurchfließen kann. Dabei ist der Durchgangsquerschnitt zwischen dem mindestens einen Durchgangsbegrenzungselement und der Aufnahme von einem Rand der Taschenöffnung und der inneren Oberfläche der Aufnahme begrenzt. Der Durchgangsquerschnitt der Tasche wird durch den Rand der Taschenöffnung und die äußere Oberfläche des Auszugskörpers begrenzt.

Der Durchgangsquerschnitt zwischen dem mindestens einen Durchgangsbegrenzungselement und der inneren Oberfläche der Aufnahme und der Durchgangsquerschnitt der Tasche können, senkrecht zur Auszugsrichtung betrachtet, unterschiedliche Größen aufweisen. Diese unterschiedlichen Durchgangsquerschnittsabmessungen der beiden möglichen Fließwege können zu einem unterschiedlichen Viskositätsverhalten des dilatanten Fluids beim Durchfließen des Durchgangsquerschnitts zwischen dem mindestens einen Durchgangsbegrenzungselement und der inneren Oberfläche der Aufnahme und des Durchgangsquerschnitts der Tasche führen.

Ist der Durchgangsquerschnitts der Tasche kleiner als der Durchgangsquerschnitt zwischen dem mindestens einen Durchgangsbegrenzungselement und der inneren Oberfläche der Aufnahme, so kann der Durchfluss des dilatanten Fluids durch die Taschenöffnung im Vergleich zu dem Durchfluss durch den Durchgangsquerschnitt zwischen dem mindestens einen Durchgangsbegrenzungselement und der inneren Oberfläche der Aufnahme vorzeitig eingeschränkt werden. Bei einer großen Auszugsgeschwindigkeit kann aufgrund von Verwirbelungen, welche auf einen Rückstau des dilatanten Fluids zurückzuführen sind, die Viskosität des dilatanten Fluids erhöht werden. Somit kann der Durchgang für das dilatante Fluid durch die Taschenöffnung blockiert werden. Das dilatante Fluid kann dann nur noch durch den Durchgangsquerschnitt zwischen dem mindestens einen Durchgangsbegrenzungselement und der inneren Oberfläche der Aufnahme fließen. Somit kann bei hohen Auszugsgeschwindigkeiten der Durchgangsquerschnitt durch die Reduktion der Anzahl der möglichen Fließwege für das dilatante Fluid verändert werden.

In einer bevorzugten Ausgestaltung ist zwischen der Aufnahme und dem Auszugskörper eine Membrane parallel zur Auszugsrichtung bereitgestellt, welche eine Einlassöffnung im Bereich eines zweiten Endes der Aufnahme zum Aufnehmen von dilatantem Fluid und im Bereich eines ersten Endes der Aufnahme eine Auslassöffnung zum Auslassen von dilatantem Fluid aufweist, wobei die Membrane das Innere der Aufnahme in eine erste Kammer zum Aufnehmen des Auszugskörpers und eine zweite Kammer teilt, wobei der Einlassöffnung ein Abstreifer vorgelagert ist, um das dilatante Fluid von der ersten Kammer durch die Einlassöffnung in die zweite Kammer zu führen.

Dadurch kann dilatantes Fluid, welches aufgrund von mindestens einem an dem Auszugskörper angeordneten mindestens einen Durchgangsbegrenzungselement in Richtung des zweiten Endes der Aufnahme befördert wird, von dem Abstreifer erfasst werden und durch die Einlassöffnung der Membrane gefördert werden. Die Membrane erstreckt sich dabei von dem ersten Ende der Aufnahme bis zu dem zweiten Ende der Aufnahme und schließt mit der inneren Oberfläche der Aufnahme ein Volumen ein.

Die zweite Kammer verläuft parallel zum Auszugskörper. Aufgrund der elastischen Eigenschaften der Membrane kann sich diese je nach Menge des dilatanten Fluids in der ersten Kammer bzw. der zweiten Kammer in eine Richtung senkrecht zur Auszugsrichtung ausdehnen.

Beim Herausziehen des Auszugskörpers aus der Aufnahme kann dieser über das mindestens eine Durchgangsbegrenzungselement dilatantes Fluid in Richtung des zweiten Endes der Aufnahme fördern. Ferner trägt auch eine Wandhaftung des dilatanten Fluids am Auszugskörper zur Förderung des dilatanten Fluids in Richtung des zweiten Endes der Aufnahme bei. Das von dem mindestens einen Durchgangsbegrenzungselement und dem Auszugskörper geförderte dilatante Fluid kann von dem der Einlassöffnung der Membrane vorgelagerten Abstreifer in die Einlassöffnung umgelenkt werden. Dadurch wird dilatantes Fluid in die zweite Kammer der Membrane gefördert.

Bevorzugt ist die Auslassöffnung kleiner als die Einlassöffnung, wodurch in der zweiten Kammer ein Staudruck gebildet wird, welcher die Membrane hin zu dem Auszugskörpers ausdehnt. Auf diese Weise wird ein Durchgangsquerschnitt zwischen dem mindestens einen Durchgangsbegrenzungselement und der Membrane in der ersten Kammer reduziert.

In einer Weiterbildung ist die Aufnahme elastisch.

Entsprechend kann beim Herausziehen des Auszugskörpers aus der Aufnahme, aufgrund des dabei entstehenden Unterdrucks in der Aufnahme, ein Einschnüren der Aufnahme senkrecht zur Auszugsrichtung erfolgen. Dabei können sich insbesondere die Aufnahmeflächen, welche parallel zum Auszugskörper verlaufen, zusammenziehen. Auf diese Weise kann der Durchgangsquerschnitt für das dilatante Fluid zwischen dem Auszugskörper und der Aufnahme reduziert werden.

In einer bevorzugten Weiterbildung umfasst die Aufnahme eine Flechtstruktur, bevorzugt einen Ziehstrumpf, welche einen Aufnahmekörper umschließt.

Dabei kann eine Flechtstruktur ein Volumen einschließen. Die Flechtstruktur kann das Volumen aufgrund einer Zugbelastung, welche durch eine unphysiologische Bewegung des Anwenders hervorgerufen wird, einschnüren bzw. komprimieren. Dies ist auf einen Kraftfluss zwischen Auszugskörper und Aufnahme zurückzuführen, welcher auf einer aus der unphysiologischen Bewegung resultierenden, gesteigerten Viskosität des dilatanten Fluids basiert.

Die Flechtstruktur der Aufnahme wird dabei in Auszugsrichtung in die Länge gezogen, wodurch es zu einer Einschnürung der Flechtstruktur quer zur Auszugsrichtung kommt. Die innere Oberfläche der Aufnahme bewegt sich auf den Auszugskörper zu und verringert dadurch den Durchgangsquerschnitt zwischen Auszugskörper und Aufnahme. Dadurch wird eine Veränderung des Durchgangsquerschnitts zwischen Auszugskörper und Aufnahme ermöglicht, welche sich über die gesamte Länge des überlappenden Bereichs von Auszugskörper und Aufnahme erstreckt.

Durch einen reduzierten Durchgangsquerschnitt zwischen Auszugskörper und Aufnahme kommt es zu einer Erhöhung der Viskosität des dilatanten Fluids und somit zu einer Einschränkung des Bewegungsumfangs der orthopädischen Vorrichtung.

Bei kraftfarmen, physiologischen Bewegungen, bei welchen nur geringe Kräfte in den Auszugskörper beziehungsweise in die Flechtstruktur eingeleitet werden, kommt es nicht zu einer Einschnürung der Flechtstruktur. Dies ist darauf zurückzuführen, dass bei physiologischen Bewegungen das dilatante Fluid nur eine geringe Viskosität aufweist und somit kein Kraftfluss oder nur ein geringer Kraftfluss zwischen dem Auszugskörper und der Aufnahme bereitgestellt wird. Der Auszugskörper und die Aufnahme gleiten vielmehr relativ zueinander aufgrund der geringen Viskosität des dilatanten Fluids, welche auf einen großen Durchgangsquerschnitt in der Ausgangsposition der orthopädischen Vorrichtung zurückzuführen ist.

Zusätzlich können zwischen der Flechtstruktur und einer äußeren Umfangsoberfläche der Aufnahme Abstandshalter angeordnet sein, wodurch verhindert werden kann, dass eine geringfügige Einschnürung der Flechtstruktur bereits Auswirkungen auf die Aufnahme hat. Entsprechend bleibt der Durchgangsquerschnitt zwischen Auszugskörper und Aufnahme solange konstant, bis die Einschnürung der Flechtstruktur sich über die Abstandshalter hinaus fortsetzt.

Beispielsweise können die Abstandshalter elastisch ausgebildet sein, bevorzugt in Form von Federelementen. Dadurch kann die Reduktion des Durchgangsquerschnitts zwischen dem Auszugskörper und der Aufnahme durch ein Zusammenziehen der Flechtstruktur bis zur Erschöpfung eines Federwegs verhindert werden.

Die elastischen Abstandshalter, beispielsweise in Form von Federelementen, haben den weiteren Vorteil, dass nach dem Wegfall einer Zugbelastung der Flechtstruktur die Flechtstruktur über die elastischen Abstandshalter wieder in ihre Ausgangsposition zurückgeführt werden kann. Die Aufnahme ist elastisch ausgebildet, um nach dem Wegfall einer Zugbelastung der Flechtstruktur die Flechtstruktur wieder in ihre Ausgangsposition, bevorzugt ihren Umfang in der Ausgangsposition, zu versetzen und um den Durchgangsquerschnitt zwischen Auszugskörper und Aufnahme, welcher in der Ausgangsposition vorgelegen hat, wieder herzustellen.

In einer weiter bevorzugten Ausführungsform ist zwischen der Aufnahme und dem Auszugskörper ein Ziehstrumpf zum abschließen der mit dilatantem Fluid gefüllten Aufnahme angeordnet, wobei ein erstes Ende des Ziehstrumpfes an einem oberen Abschnitt des Auszugskörpers umfänglich befestigt ist, und ein zweites Ende des Ziehstrumpfes an einem Öffnungsrand der Aufnahme umlaufend befestigt ist.

Dabei ist der Ziehstrumpf an einem ersten Ende derart mit dem Auszugskörper verbunden, dass das erste Ende des Ziehstrumpfs den Längsbewegungen des Auszugskörpers in Auszugsrichtung und entgegengesetzt der Auszugsrichtung folgt.

An einem zweiten Ende ist der Ziehstrumpf mit dem Öffnungsrand der Öffnung der Aufnahme verbunden. Entsprechend sitzt der Ziehstrumpf wie ein Deckel auf der Aufnahme und grenzt das Volumen, in welchem das dilatante Fluid aufgenommen ist, gegenüber der Umgebung ab.

Wird der Auszugskörper aus der Aufnahme herausgezogen, wodurch das erste Ende des Ziehstrumpfs sich von dem zweiten Ende des Ziehstrumpfs entfernt, beginnt sich die innere Oberfläche des Ziehstrumpfs auf den Auszugskörper zuzubewegen. Ein Durchgangsquerschnitt, welcher senkrecht zur Auszugsrichtung zwischen dem Auszugskörper und einer inneren Oberfläche des Ziehstrumpfs liegt, kann dadurch verringert werden.

Je weiter der Auszugskörper aus der Aufnahme herausgezogen wird, je weiter verringert sich der Durchgangsquerschnitt zwischen dem Auszugskörper und der inneren Oberfläche des Ziehstrumpfs. Dadurch erhöht sich die Viskosität des dilatanten Fluids im Bereich zwischen dem Auszugskörper und der inneren Oberfläche des Ziehstrumpfs, was zu einer Einschränkung der Bewegungsmöglichkeit der orthopädischen Vorrichtung führt.

In einer weiter bevorzugten Ausgestaltung ist das mindestens eine Durchgangsbegrenzungselement einen Torsionskörper, welcher um die Längsachse des mindestens einen Durchgangsbegrenzungselements von einer Ausgangsposition in eine Begrenzungsposition und von der Begrenzungsposition in die Ausgangsposition verdrehbar ist, wobei in einer Ausgangsposition eine schmale Seite des mindestens einen Durchgangsbegrenzungselements in eine Auszugsrichtung zeigt und in einer Begrenzungsposition eine breite Seite des mindestens einen Durchgangsbegrenzungselements in die Auszugsrichtung zeigt.

Dadurch kann das mindestens eine Durchgangsbegrenzungselement eine Torsion erfahren. Ursache für die Torsion sind die durch die Auszuggeschwindigkeit hervorgerufenen Kräfte, welche auf das mindestens eine Durchgangsbegrenzungselement wirken. Die Verdrehung des mindestens einen Durchgangsbegrenzungselements kann durch ein Torsionsmoment, welches beim Ausziehen des Auszugskörpers durch eine in Auszugsrichtung gesehene asymmetrische Geometrie des mindestens einen Durchgangsbegrenzungselements und einer daraus resultierenden asymmetrischen Anströmung, hervorgerufen werden. So kann das mindestens eine Durchgangsbegrenzungselement entlang seiner Längsachse bereits im Ausgangszustand eine leichte Verdrehung aufweisen und um eine Krümmungsachse, welche senkrecht zur Längsachse des mindestens einen Durchgangsbegrenzungselement steht, gekrümmt sein. Es können aber auch andere Möglichkeiten zur Erzeugung des Torsionsmoments auf das mindestens eine Durchgangsbegrenzungselement genutzt werden, wie beispielsweise mindestens ein einseitig angebrachter Vorsprung im Bereich eines zweiten Endes des mindestens einen Durchgangsbegrenzungselements.

Wird das mindestens eine Durchgangsbegrenzungselement um einen Verdrehwinkel tordiert, so weist die breite Seite des mindestens einen Durchgangsbegrenzungselements nun entsprechend dem Betrag des Verdrehwinkels in Auszugsrichtung. Hierdurch ist zum einen die projizierte Fläche in Auszugsrichtung des mindestens einen Durchgangsbegrenzungselements vergrößert, was einen vergrößerten Auszugswiderstand und eine vermehrte Bildung von Turbulenzen und Wirbel zur Folge hat. Die projizierte Fläche entsteht, wenn man das mindestens eine Durchgangselement parallel zur Auszugsrichtung auf eine Fläche senkrecht zur Auszugsrichtung projiziert. Zum anderen ist der Abstand zwischen der inneren Oberfläche der Aufnahme und dem mindestens einen Durchgangsbegrenzungselement reduziert, was einen geringeren Durchgangsquerschnitt generiert. Mit zunehmendem Verdrehwinkel des mindestens einen Durchgangsbegrenzungselements steigen hierbei der Auszugswiderstand und aufgrund des verkleinerten Durchgangsquerschnitts ebenfalls die Scherrate und somit die Viskosität des dilatanten Fluids. Der Verdrehwinkel steigt mit zunehmender Auszugsgeschwindigkeit entsprechend an.

Aufgrund dieses Wirkprinzips und den dadurch variierenden Kräften erfährt das mindestens eine Durchgangsbegrenzungselement je nach Auszugsgeschwindigkeit eine unterschiedlich starke Verdrehung.

Durch entsprechende Auslegungen des mindestens einen Durchgangsbegrenzungselements kann eine Wirkgeometrie erzielt werden, durch welche sich bei physiologischer Bewegung und entsprechend geringer Auszugsgeschwindigkeit das mindestens eine Durchgangsbegrenzungselement nicht verdrehen und der Durchgangsquerschnitt nicht verändern kann. Bei unphysiologischer Bewegung und entsprechend hoher Auszugsgeschwindigkeit hingegen, erfährt das mindestens eine Durchgangsbegrenzungselement durch die von dem umströmenden Fluid hervorgerufenen Kräfte ein entsprechend großes Torsionsmoment, was zu einer starken Verdrehung des mindestens einen Durchgangsbegrenzungselements führt. Diese Verdrehung führt zu einer Verengung des Durchgangsquerschnitts und damit zu einer Erhöhung der Viskosität.

In einer weiteren Ausführungsform weist die Aufnahme an einem Ende eine Ausgleichsmembran auf, welche eine durch eine Bewegung des Auszugkörpers hervorgerufene Volumenänderung innerhalb der Aufnahme ausgleichen kann. Dadurch kann beim Ausziehen der Auszugskörpers ein Vakuum- Effekt vermieden werden, welcher sich nachteilig auf die Viskosität des dilatanten Fluids auswirken und überdies eine zusätzliche Rückhaltekraft auf den Auszugskörper hervorrufen kann. Mit anderen Worten kommt es durch die Ausgleichsmembran zu einem Druckausgleich innerhalb der Aufnahme. Dadurch kann insbesondere im Bereich physiologischer Bewegungen im Vergleich zu einer starren Aufnahme eine nochmals erhöhte Leichtgängigkeit der orthopädischen Vorrichtung bereitgestellt werden.

In einer Weiterbildung beträgt der Abstand zwischen dem Auszugskörper und der Aufnahme senkrecht zur Auszugskörperrichtung in der Ausgangsposition zwischen 0,01 und 1000 mm, bevorzugt 0,1 bis 50 mm, besonders bevorzugt 0,1 bis 15 mm, und ganz besonders bevorzugt 0,1 bis 5 mm.

Der Abstand kann zum einen auf die Eigenschaften des verwendeten dilatanten Fluids und zum anderen auf das Einsatzgebiet der Orthopädischen Vorrichtung abgestimmt werden.

In einer weiteren Ausführungsform weist das mindestens eine Durchgangsbegrenzungselement Kautschuk oder Kunststoff, bevorzugt Silicon oder thermoplastischem Kunststoff, besonders bevorzugt Polypropylen, Polyethylen und/oder Polyurethan auf.

Dadurch können dem mindestens einen Durchgangsbegrenzungselement die nötige Festigkeit und Steifigkeiten verliehen werden zum anderen lassen sich aber auch elastische Eigenschaften des mindestens einen Durchgangsbegrenzungselements auf der Grundlage dieser Materialien realisieren.

In einer weiter bevorzugten Ausführungsform umfasst das dilatante Fluid eine Dispersion aus Ethylenglykol oder Silikon-Öl und Siliciumdioxid, bevorzugt Silica Gel mit einer Teilchengröße von 2 bis 1000 nm, Oberflächen von 30 bis 250 m²/g, einen Feststoffanteil von 5 bis 80 Gew.-% und Stabilisatoren.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1: schematisch eine Seitenansicht im Querschnitt durch eine orthopädischen Vorrichtung in einer Ausgangsposition;
- Figur 2: schematisch eine Seitenansicht im Querschnitt durch die orthopädischen Vorrichtung in einer von der Ausgangsposition abweichenden Position;
- Figur 3a: schematisch eine Draufsicht in einer Schnittdarstellung der orthopädischen Vorrichtung aus Figur 2;
- Figur 3b: schematisch eine Draufsicht in einer Schnittdarstellung der orthopädischen Vorrichtung aus Figur 3b;
- Figur 4: schematisch eine Detailansicht im Seitenschnitt eines eine Aussparung aufweisenden Auszugskörpers;
- Figur 5: schematisch eine Seitenansicht im Querschnitt einer orthopädischen Vorrichtung mit einem Durchgänge aufweisenden Auszugskörper in einer Ausgangsposition;
- Figur 6: schematisch eine Seitenansicht im Querschnitt der orthopädischen Vorrichtung aus Figur 5 in einer von der Ausgangsposition abweichenden Position;
- Figur 7: schematisch eine Vorderansicht eines Auszugskörpers mit Durchgängen und darin befindlichen Durchgangsbegrenzungselementen;
- Figur 8: schematisch eine Seitenansicht im Querschnitt einer orthopädischen Vorrichtung mit einem Auszugskörper gemäß Figur 5 mit einem Vorsprung am Durchgangsbegrenzungselement in einer Ausgangsposition;
- Figur 9: schematisch eine Seitenansicht im Querschnitt der orthopädischen Vorrichtung gemäß Figur 7 mit einem Vorsprung am Durchgangsbegrenzungselement;
- Figur 10: schematisch eine Seitenansicht im Querschnitt durch eine orthopädischen Vorrichtung mit einem ankerförmigen Durchgangsbegrenzungselement in einer Ausgangsposition;
- Figur 11: schematisch eine Seitenansicht im Querschnitt durch die orthopädischen Vorrichtung gemäß Figur 10 in einer von der Ausgangsposition abweichenden Position;
- Figur 12a: schematisch eine Vorderansicht im Querschnitt durch eine orthopädischen Vorrichtung mit einem Durchgangsbegrenzungselement in Form eines Torsionskörpers in einer Ausgangsposition;
- Figur 12b: schematisch eine Draufsicht in einer Schnittdarstellung der orthopädischen Vorrichtung aus Figur 12a;
- Figur 13a: schematisch eine Vorderansicht im Querschnitt durch die orthopädische Vorrichtung gemäß Figur 12a in einer von der Ausgangsposition abweichenden Position;
- Figur 13b: schematisch eine Draufsicht in einer Schnittdarstellung der orthopädischen Vorrichtung aus Figur 13a;
- Figur 14: schematisch eine Seitenansicht im Querschnitt durch eine orthopädischen Vorrichtung mit einem Durchgangsbegrenzungselement in Taschenform in einer Ausgangsposition;
- Figur 15: schematisch eine Seitenansicht im Querschnitt durch die orthopädischen Vorrichtung gemäß Figur 14 in einer von der Ausgangsposition abweichenden Position;
- Figur 16: schematisch eine Seitenansicht im Querschnitt durch eine orthopädischen Vorrichtung mit einer Membrane in einer Ausgangsposition;
- Figur 17: schematisch eine Seitenansicht im Querschnitt durch die orthopädischen Vorrichtung gemäß Figur 16 in einer von der Ausgangsposition abweichenden Position;
- Figur 18: schematisch eine Seitenansicht im Querschnitt durch eine orthopädischen Vorrichtung mit einer Ausgleichsmembran an einem Ende der Aufnahme in einer Ausgangsposition;
- Figur 19: schematisch eine Seitenansicht im Querschnitt durch eine orthopädischen Vorrichtung mit einer Ausgleichsmembran an einem Ende der Aufnahme in einer von der Ausgangsposition abweichenden Position;
- Figur 20a: schematisch eine Vorderansicht im Querschnitt durch eine orthopädischen Vorrichtung mit einem Durchgangsbegrenzungselement in Form eines Schirms in einer Ausgangsposition;
- Figur 20b: schematisch eine Draufsicht in einer Schnittdarstellung der orthopädischen Vorrichtung aus Figur 20a;
- Figur 21a: schematisch eine Vorderansicht im Querschnitt durch die orthopädische Vorrichtung gemäß Figur 20a in einer von der Ausgangsposition abweichenden Position;
- Figur 21b: schematisch eine Draufsicht in einer Schnittdarstellung der orthopädischen Vorrichtung aus Figur 21a;
- Figur 22: schematische Darstellung des Verlaufs der Viskosität über die jeweils zugehörige Scherrate in Bezug auf unterschiedliche Durchgangsquerschnitte;
- Figur 23: schematisch eine Sprunggelenksorthese mit einer orthopädischen Vorrichtung; und
- Figur 24: schematisch eine Handgelenksorthese mit einer orthopädischen Vorrichtung.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf eine wiederholte Beschreibung dieser Elemente in der nachfolgenden Beschreibung teilweise verzichtet.

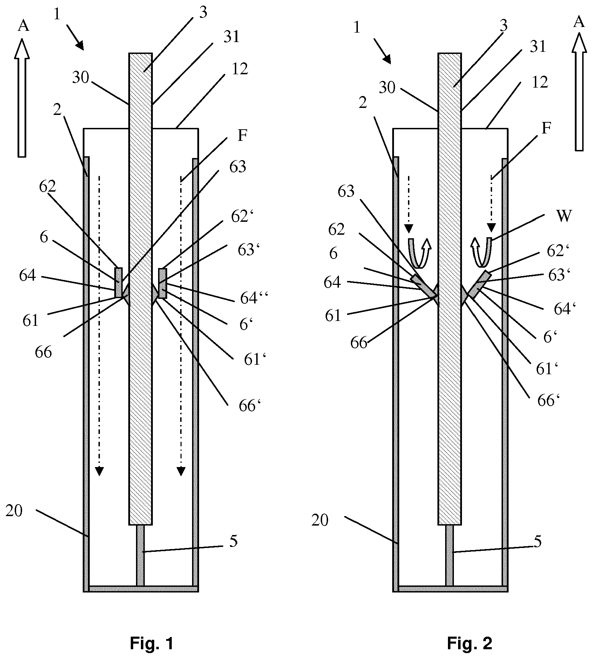

Figur 1 zeigt schematisch eine orthopädische Vorrichtung 1 zur Begrenzung der Bewegung eines zwischen einem ersten und einem zweiten Körperbereich angeordneten Gelenks. Dabei umfasst die Vorrichtung 1 eine mit einem dilatanten Fluid gefüllte Aufnahme 2, welche an einem ersten Körperbereich fixiert werden kann, und einen Auszugskörper 3, welcher an einem zweiten Körperbereich fixiert werden kann, in einer Auszugsrichtung A relativ zur Aufnahme 2 beweglich ist und sich zum Teil in das Innere der Aufnahme 2 erstreckt, so, dass zwischen der Aufnahme 2 und dem Auszugskörper 3 ein mit dem dilatanten Fluid gefüllter Durchgangsquerschnitt bereitgestellt ist.

Im vorliegenden Fall ist der Auszugskörper 3 aus Polyamid und die Aufnahme 2 aus Polyurethan hergestellt. Alternativ können die beiden Bauteile auch aus anderen Kunststoffen, wie Silikon, Gummi oder bevorzugt einem anderen thermoplastischen Material wie beispielsweise Beispiel Polyamid, Polypropylen, Polyethylen oder Polyurethan bestehen, wobei insbesondere bei der Aufnahme 2 darauf zu achten ist, dass das verwendete Material fluidundurchlässig ist.

Der Auszugskörper 3 weist in der in Figur 1 gezeigten Variante auf einer Vorderseite 30 und einer Rückseite 31 jeweils ein Durchgangsbegrenzungselement 6, 6' auf. Das Durchgangsbegrenzungselement 6, 6' weist eine erste Oberfläche 63, 63' auf, welche der Auszugskörperoberfläche zugewandt ist, und eine zweite Oberfläche 64, 64', welche der inneren Oberfläche 20 der Aufnahme 2 zugewandt ist. Des Weiteren umfasst das Durchgangsbegrenzungselement 6, 6' ein erstes Ende 61, 61', welches mit dem Auszugskörper 3 verbunden ist, und ein zweites Ende 62, 62', welches freistehend ist und in Richtung der Auszugsrichtung A weist. In Figur 1 ist das Durchgangsbegrenzungselement 6, 6' an seinem ersten Ende 61, 61' über ein Gelenk 66, 66', hier in Form eines Filmscharniers, mit dem Auszugskörper 3 verbunden. Das Filmscharnier ist in diesem Fall als eine lokale Reduktion der Wandstärke des Durchgangsbegrenzungselements 6, 6' bereitgestellt. Hierdurch ist die Biegesteifigkeit des Durchgangsbegrenzungselements 6, 6' in dem Bereich des Filmscharniers reduziert. Somit ist das Durchgangsbegrenzungselement 6, 6' um das Gelenk 66, 66', in diesem Fall dem Filmscharnier, relativ zum Auszugskörper 3 klappbar angeordnet. In der Ausgangsposition ist das Durchgangsbegrenzungselement 6, 6' parallel zum Auszugskörper 3, folglich in Auszugsrichtung A ausgerichtet. Die beiden Durchgangsbegrenzungselemente 6, 6' sind bezogen auf die Auszugsrichtung A am Auszugskörper 3 auf gleicher Höhe angebracht. Hierdurch entstehen beim Ausziehen des Auszugskörpers 3 aus der Aufnahme 2 auf der Vorderseite 30 und der Rückseite 31 des Auszugskörpers 3 symmetrische Strömungsverhältnisse des dilatanten Fluids beziehungsweise eine symmetrische Fluidströmung F relativ zum Auszugskörper 3 und somit eine symmetrische Lastverteilung innerhalb der orthopädischen Vorrichtung 1. Alternativ können die Durchgangsbegrenzungselemente 6, 6' auch versetzt angeordnet sein.

Das Durchgangsbegrenzungselement 6, 6' in Figur 1 ist so gestaltet, dass es selbst bei einer Auslenkung um 90 Grad zur Auszugsrichtung A die innere Oberfläche 20 der Aufnahme 2 nicht berührt. Die bei Auslenkung des Durchgangselements 6, 6' im Filmscharnier entstehenden Biegespannungen bewirken, dass sich das Durchgangsbegrenzungselement 6, 6' im lastfreien Zustand stets in die Ausgangsposition zurückstellt. Durch die Gestaltung des Filmscharniers ist es dem Durchgangsbegrenzungselement 6, 6' nicht möglich, einen Winkel größer 90 Grad zur Auszugsrichtung A einzunehmen. So kann ein Anschlag am Gelenk 66, 66' zur Begrenzung der Bewegung des Durchgangsbegrenzungselements 6, 6' bereitgestellt werden. Ebenfalls kann ein Mittel zur Rückstellung des Durchgangsbegrenzungselements 6, 6' beziehungsweise zur Erhöhung des Auslenkwiderstands um das Gelenk 66, 66', beispielsweise eine Biegefeder, bereitgestellt werden. In einer weiteren Variante kann das Durchgangsbegrenzungselement 6, 6' derart gestaltet sein, dass das zweite Ende 62, 62' bei einer maximalen Auslenkung die innere Oberfläche 20 der Aufnahme 2 berührt und hierdurch der maximale Winkel der Auslenkung des Durchgangsbegrenzungselements 6, 6' definiert wird.

In einer alternativen Ausführungsform kann das Durchgangsbegrenzungselement 6, 6' auch direkt, folglich ohne eine gelenkige Verbindung, mit dem Auszugskörper 3 verbunden sein. Alternativ kann der Auszugskörper 3 auch mehrere Durchgangsbegrenzungselemente 6, 6' auf seiner Vorderseite 30 und seiner Rückseite 31 enthalten, die zumindest in einem Bereich des Auszugskörpers 3 bevorzugt regelmäßig angeordnet sind. Besonders bevorzugt weist der Auszugskörper 3 dabei auf der Vorderseite 30 und der Rückseite 31 die gleiche Anzahl an Durchgangsbegrenzungselementen 6, 6' auf. Im vorliegenden Fall ist das Durchgangsbegrenzungselement 6, 6' aus dem gleichen Material, wie der Auszugskörper 3. Alternativ kann das Durchgangsbegrenzungselement 6, 6' aber auch aus einem anderen Material, bevorzugt einem anderen thermoplastischen Kunststoff bestehen und bevorzugt mittels eines Mehrkomponenten- Fertigungsverfahrens mit dem Auszugskörper 3 verbunden worden sein.

In der in Figur 1 gezeigten Variante der orthopädischen Vorrichtung 1 ist der Auszugskörper 3 in der Aufnahme 2 in einer nicht gezeigten Schienenführung linear bewegbar gehalten. Dabei kann der Auszugskörper 3 in Richtung der Auszugsrichtung A in die Aufnahme 2 hinein beziehungsweise aus dieser ausgefahren werden. Alternativ kann der Auszugskörper 3 auch als Abstandshalter ausgebildete lokale Erhöhungen auf seiner Oberfläche aufweisen, um den Auszugskörper 3 definiert in der Aufnahme 2 zu führen.

In der in Figur 1 gezeigten Ausführungsform weist die orthopädische Vorrichtung 1 ein Mittel zur Rückstellung 5 des Auszugskörpers 3 auf, welches nach einer Bewegung des Auszugskörpers 3 aus der Ausgangsposition in eine von dieser abweichenden Position den Auszugskörper 3 zurück in die Ausgangsposition bewegt. Abhängig von den physiologischen Bedingungen des zwischen dem ersten und dem zweiten Körperbereich angeordneten Gelenks kann das Mittel zur Rückstellung 5 notwendig sein, um das Gelenk, nachdem es eine Auslenkung erfahren hat, wieder in eine Ruheposition zurückzuführen. Dabei ist das Mittel zur Rückstellung 5 derart gewählt, dass das Gelenk eine schonende Rückstellung erfährt beziehungsweise dass der Auszugskörper 3 so langsam durch das dilatante Fluid geführt wird, dass kein erheblicher Anstieg der Viskosität in dem dilatanten Fluid hervorgerufen wird. Alternativ kann das Mittel zur Rückstellung 5 auch derart ausgeführt sein, dass es lediglich zur Rückstellung des Auszugskörpers 3 in die Ausgangsposition vorgesehen ist, und keinen Einfluss auf die Gelenkstellung hat. Somit ermöglicht das Mittel zur Rückstellung 5 in diesem Fall, dass der Auszugskörper 3, welcher beispielsweise durch eine Elongation des Gelenks einen Positionswechsel in eine von der Ausgangsposition abweichenden Position um einen Versatz in Auszugrichtung A verschoben wurde, nach einer Rückstellung des Gelenks in seine Ruhelage ebenfalls wieder in seine Ausgangsposition zurückkehrt.

Das in Figur 1 gezeigte Mittel zur Rückstellung 5 ist ein elastischer Kunststoff in Form eines Gummibandes mit den oben beschriebenen Eigenschaften. Alternativ kann das Mittel zur Rückstellung 5 auch durch eine Feder, durch ein Paar aus Permanentmagneten, wobei der eine Permanentmagnet am unteren Ende der Aufnahme 2 und der andere Permanentmagnet an dem den unteren Ende der Aufnahme 2 zugewandten Ende des Auszugskörpers 3 angeordnet ist, oder durch eine elastische Dichtung ausgebildet sein. Darüber hinaus kann die Rückstellung des Auszugskörpers 3 auch über einen in der Aufnahme 2 herrschenden Unterdruck erfolgen. Der Unterdruck entsteht dabei beim Herausziehen des Auszugskörpers 3 entlang der Auszugsrichtung A.

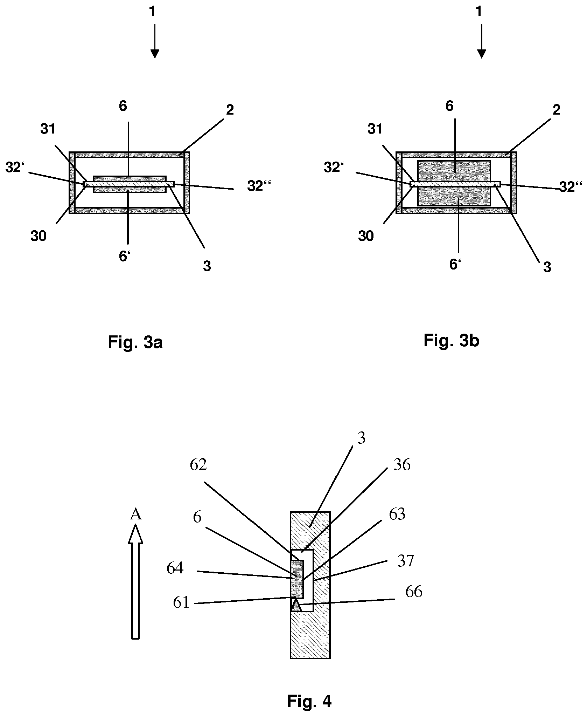

Darüber hinaus ist der Figur 1 schematisch eine Dichtung 12 zu entnehmen, welche am oberen, zu einer äußeren Umgebung offenen Ende der Aufnahme 2 sitzt und den Innenraum der Aufnahme 2 gegen die äußere Umgebung abdichtet. Die Dichtung 12 umläuft dabei die Vorderseite 30, die Rückseite 31 sowie die Seitenflächen 32', 32" (siehe Figur 3) des Auszugskörpers 3. Dabei kann der Auszugskörper 3 relativ zur Dichtung 12 bewegt werden. Eine umlaufende Dichtlippe der Dichtung 12 verhindert dabei, dass dilatantes Fluid aus dem Inneren der Aufnahme 2 austritt. Im vorliegenden Fall besteht die Dichtung aus thermoplastischem Polyurethan, alternativ kann die Dichtung aber auch aus einem anderen Kunststoff, wie beispielsweise Latex, bestehen.

Alternativ kann die Dichtung 12 auch balgförmig ausgebildet sein. Dabei ist ein erstes Ende des Dichtungsbalges fest mit der Aufnahme 2 und ein zweites Ende des Dichtungsbalges fest mit dem Auszugskörper 3 verbunden. Wird der Auszugskörper 3 aus der Aufnahme 2 heraus bewegt, so vergrößert sich der Abstand zwischen dem ersten und dem zweiten Ende des Dichtungsbalges. Entsprechend folgt der Dichtungsbalg den Bewegungen der orthopädischen Vorrichtung 1 beziehungsweise des Auszugskörpers 3.

Darüber hinaus kann eine Balgdichtung zusätzlich die Funktion des Mittels zur Rückstellung übernehmen. In diesem Fall weist der Balgkörper der Dichtung 12 eine Elastizität auf, die es ermöglicht, nach Auslenkung der orthopädischen Vorrichtung 1 wieder eine Ruheposition einzunehmen, indem sich das erste Ende und das zweite Ende der Balgdichtung wieder einander annähern. Als Dichtmaterial kommt dabei bevorzugt ein elastischer Gummi zum Einsatz.

Wird der Auszugskörper 3 in die Aufnahme 2 hinein oder aus der Aufnahme 2 heraus bewegt, so wird die Bewegung des Auszugskörpers 3 von den Eigenschaften des dilatanten Fluides beeinflusst. Eine Bewegung des Auszugskörpers 3 relativ zur Aufnahme 2 generiert aufgrund der Wandhaftung zwischen dem dilatanten Fluid und den Auszugskörperoberflächen (30, 31) beziehungsweise der Inneren Oberfläche 20 der Aufnahme 2 eine Scherkrafteinleitung über diese Oberflächen in das dilatante Fluid. Aufgrund der Viskosität des dilatanten Fluids kann somit eine Kraft in Richtung der Auszugsrichtung A über Scherung des dilatanten Fluids vom Auszugskörper 3 auf die Aufnahme 2 übertragen werden. Die Höhe der zu übertragenden Kräfte ist hierbei von der Geschwindigkeit des Auszugskörpers 3 relativ zur Aufnahme 2 sowie in hohem Maße vom Durchgangsquerschnitt abhängig.

Wird die orthopädische Vorrichtung 1 folglich unter einer geringen Krafteinwirkung beziehungsweise mit einer physiologischen Geschwindigkeit ineinander beziehungsweise auseinander geschoben, so kommt es aufgrund der niedrigen Viskosität des dilatanten Fluids lediglich zu einer geringen Scherkrafterzeugung, somit ist der Auszugskörper 3 in dem dilatanten Fluid sehr leicht zu bewegen.

Bei einer Auszugsbewegung werden die Durchgangsbegrenzungselemente 6, 6' von dem dilatanten Fluid umströmt, wobei sie derart ausgebildet sind, dass sie sich aufgrund der durch die Umströmung entstehenden Kräfte auf die Durchgangsbegrenzungselemente 6, 6' bei physiologischen Auszugsgeschwindigkeiten nicht oder nur in sehr geringem Maße aus ihrer Ausgangsposition bewegen, die Lage der Durchgangsbegrenzungselemente 6, 6' entspricht somit im Wesentlichen der der Ausgangsposition.

Figur 2 zeigt die orthopädische Vorrichtung 1 aus Figur 1 in einer von der Ausgangsposition abweichenden Position. Hier ist das Durchgangsbegrenzungselement 6, 6' in Bezug auf die Ausgangsposition um das Gelenk 66, 66' in Richtung der Inneren Oberfläche 20 der Aufnahme 2 ausgelenkt, das zweite Ende 62, 62' des Durchgangsbegrenzungselements 6, 6' ist somit im Vergleich zur Ausgangsposition näher an der Aufnahmeinnenwand. Hierdurch ist der Durchgangsquerschnitt in den Bereichen des Durchgangsbegrenzungselements 6, 6' im Vergleich zur Ausgangsposition reduziert. Folglich ist die Scherrate des dilatanten Fluids im Vergleich zur Scherrate des dilatanten Fluids in der Ausgangsposition im Bereich des Durchgangsbegrenzungselements 6, 6'wesentlich höher, was bei einer Bewegung des Auszugskörpers 3 in Auszugsrichtung A zu einer Zunahme der Viskosität des dilatanten Fluids in diesen Bereichen führt und dadurch dort die entstehenden Scherkräfte im dilatanten Fluid im Vergleich zum nicht ausgelenkten Durchgangsbegrenzungselement 6, 6' wesentlich höher sind.

Das Auslenken der Durchgangsbegrenzungselemente 6, 6' entsteht bei Auszugsgeschwindigkeiten oberhalb des physiologischen Bereichs aufgrund der durch den Strömungswiderstand der Durchgangsbegrenzungselemente 6, 6' im dilatanten Fluid hervorgerufenen strömungsdynamischen Druckkräfte auf die Durchgangsbegrenzungselemente 6, 6'. Aufgrund der Geometrie der Durchgangsbegrenzungselemente 6, 6' werden diese um die Gelenke 66, 66' in Richtung der Inneren Oberfläche 20 der Aufnahme 2 geklappt.

Hierbei gilt, dass je größer die auf die orthopädische Vorrichtung 1 wirkenden Kräfte beziehungsweise je höher die Geschwindigkeiten sind, welche die Aufnahme 2 und den Auszugskörper 3 ineinander beziehungsweise auseinander bewegen, umso größer sind die zwischen dem Auszugskörper 2 und dem dilatanten Fluid auftretenden Scherkräfte. Mit dem Anstieg der Scherkräfte steigt auch die Viskosität des dilatanten Fluids. Dabei weist das dilatante Fluid einen diskreten Dilatanzsprung auf, bei welchem sprunghaft eine deutliche Zunahme der Viskosität zu verzeichnen ist. So lassen sich die Aufnahme 2 und der Auszugskörper 3 der orthopädischen Vorrichtung 1 bei einer in dem dilatanten Fluid herrschenden hohen Viskosität, insbesondere jenseits des Dilatanzsprungs, nicht mehr oder nur noch geringfügig gegeneinander bewegen beziehungsweise sind für eine weitere Bewegung sehr hohe Kräfte erforderlich.

Befinden sich die Durchgangsbegrenzungselemente 6, 6' in ausgelenkter Position, so ist die Scherrate des dilatanten Fluids in den Bereichen der Durchgangsbegrenzungselemente 6, 6' bei unphysiologischen Auszugsgeschwindigkeiten oberhalb der kritischen Scherrate, bei welchem der Dilatanzsprung eintritt, was eine sehr hohe Viskosität des dilatanten Fluids bedingt, somit ein hoher Grad der Kraftübertragung zwischen der Auszugskörper 3 und der Aufnahme 2 generiert wird und hierdurch eine hohe Rückhaltekraft entsteht, die entgegen einer den Auszug des Auszugskörpers 3 bedingende Auszugskraft wirkt. Die Bewegung des Auszugskörpers 3 und somit die Bewegung des ersten und des zweiten Körperteils, zwischen welchen die orthopädische Vorrichtung 1 angebracht ist, wird somit gebremst beziehungsweise gänzlich gehemmt.

Die ausgeklappten Durchgangsbegrenzungselemente 6, 6' bewirken im Vergleich zur Ausgangsposition aufgrund der stärkeren Umlenkung des dilatanten Fluids und des höheren Strömungswiderstand der Durchgangsbegrenzungselemente 6, 6' eine Zunahme von Turbulenzen und Wirbeln W im dilatanten Fluid, was ebenfalls zu einer Steigerung der oben beschriebenen Rückhaltekraft führt.

In einer alternativen Ausgestaltung der orthopädischen Vorrichtung können der oder die Durchgangsbegrenzungselemente statt auf dem Auszugskörper auch auf der der inneren Oberfläche der Aufnahme bereitgestellt werden. Hier ist die Orientierung der Durchgangsbegrenzungselemente in Bezug auf die Auszugsrichtung umgekehrt. Mit anderen Worten weist das zweite Ende der Auszugrichtung entgegen. Alternativ können der oder die Durchgangsbegrenzungselemente auch auf einer Seite des Auszugskörpers, beispielsweise der Vorderseite, bereitgestellt werden und die zweite Seite, hier entsprechend die Rückseite, ohne Durchgangsbegrenzungselemente ausgeführt sein. Auf dem Teil der inneren Oberfläche der Aufnahme, welche der zweiten Seite, hier entsprechend die Rückseite, gegenüber liegt, mit einem oder mehreren Durchgangsbegrenzungselementen versehen sein. Möglich ist auch eine Kombination aus Durchgangsbegrenzungselementen auf der inneren Oberfläche der Aufnahme und dem Auszugskörper.

Figur 3a zeigt schematisch eine Draufsicht in einer Schnittdarstellung der orthopädischen Vorrichtung 1 aus Figur 1. Die Durchgangsbegrenzungselemente 6, 6' befinden sowohl auf der Vorderseite 30, als auch auf der Rückseite 31 des Auszugskörpers 3 in der Ausgangsposition, folglich in einem eingeklappten Zustand. Die Vorderseite 30 und die Rückseite 31 des Auszugskörpers 3 sind um ein Vielfaches größer, als die beiden Seitenflächen 32', 32" des Auszugskörpers 3. Somit sind die Vorderseite 30 und die Rückseite 31 und die darauf befindlichen Durchgangsbegrenzungselemente 6, 6' für den Großteil der Scherkraftübertragung von dem Auszugskörper 3 auf das dilatante Fluid verantwortlich.

Im Vergleich zeigt Figur 3b schematisch eine Draufsicht in einer Schnittdarstellung der orthopädischen Vorrichtung 1 aus Figur 2. Deutlich zu erkennen ist der durch die ausgeklappte Position der Durchgangselemente 6 resultierende stark verkleinerte Durchgangsquerschnitt. Durch die ausgeklappte Position der Durchgangsbegrenzungselemente 6 ist weiterhin der Auszugswiderstand in Auszugsrichtung A aufgrund der vergrößerten projizierten Fläche der Durchgangsbegrenzungselemente 6 vergrößert. Bei einer Bewegung der Auszugskörpers 3 in Auszugsrichtung erfährt das dilatante Fluid somit eine stärkere Umlenkung, des Weiteren kommt es zu einer vermehrten Bildung von Turbulenzen und Wirbeln W.

Figur 4 zeigt schematisch eine Detailansicht im Seitenschnitt eines eine Aussparung 36 aufweisenden Auszugskörpers 3. Hier weist die erste Oberfläche 63 des Durchgangsbegrenzungselements 6 einen Abstand zu einer Innenfläche 37 der Aussparung 36 auf, damit das dilatante Fluid während einer Auszugsbewegung das Durchgangsbegrenzungselement 6 auf der ersten Oberfläche 63 umströmen und hierdurch bei unphysiologischen Auszugsgeschwindigkeiten eine Auslenkung des Durchgangsbegrenzungselements 6 bewirken kann. Im Fall, dass der Auszugkörper 3 mehrere Aussparungen 36 aufweist, können die Aussparungen 36 des Auszugskörpers 3 mit dem darin enthaltenen Durchgangsbegrenzungselementen 6 entsprechend dem Auszugskörper 3 aus Figur 1 und 2 bevorzugt ebenfalls auf der Vorderseite 30 und der Rückseite 31 des Auszugskörpers 3 vorgesehen sein. Durch das Versenken der Durchgangsbegrenzungselemente 6 in die Aussparungen 36 kann der gesamte Abstand des Auszugskörpers 3 zur Aufnahme senkrecht zur Auszugsrichtung A als Durchgangsquerschnitt für das dilatante Fluid genutzt werden. Durch die für das zu schonende Gelenk unkritischen Krafteinwirkungen auf die orthopädische Vorrichtung 1 können Auszugskörper 3 und Aufnahme relativ zueinander bewegt werden, wobei der Durchgangsquerschnitt nicht von den Durchgangsbegrenzungselementen 6 beeinflusst beziehungsweise reduziert wird.

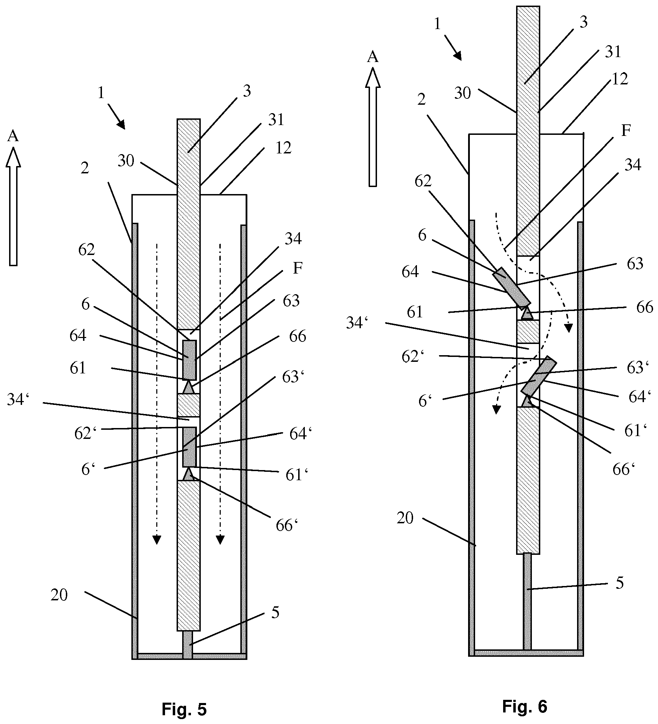

Figur 5 zeigt schematisch eine Seitenansicht im Querschnitt einer alternativen Variante einer orthopädischen Vorrichtung gemäß Figur 1 in einer Ausgangsposition. Der Auszugskörper 3 weist Durchgänge 34, 34' auf, welche den Auszugskörper 3 von der Vorderseite 30 bis zur Rückseite 31 komplett durchdringen. Die Durchgangsbegrenzungselemente 6, 6' befinden sich hierbei in den Durchgängen 34, 34' des Auszugkörpers 3 und sind mittels Gelenken 66 , 66' mit dem Auszugskörper 3 verbunden, wobei die Durchgangsbegrenzungselemente 6, 6' parallel zur Auszugsrichtung A ausgeprägt sind, wodurch es zu keiner lokalen Verkleinerung des Durchgangsquerschnitts kommt, wie dies bei der Anordnung der Durchgangsbegrenzungselemente 6, 6' in Figur 1 der Fall ist. Insgesamt lassen sich dadurch geringe Bauteilhöhen der orthopädischen Vorrichtung 1 realisieren. So bedarf es keines zusätzlichen Abstands zwischen Auszugskörper 3 und Aufnahme 2 für die Bereitstellung der Durchgangsbegrenzungselemente 6, 6'. Aufgrund der geringen Strömungsumlenkung der Fluidströmung F bei Bewegungen des Auszugskörpers 3 mit Durchgangsbegrenzungselementen 6, 6' dieser Geometrie werden ebenfalls weniger viskositätserhöhende Turbulenzen und Wirbel W hervorgerufen, was eine hohe Leichtgängigkeit der Bewegung des Auszugskörpers 3 in Auszugsrichtung A ermöglicht.

Bei der vorliegenden Variante sind die Durchgangsbegrenzungselemente 6, 6' mittig in den Durchgängen 34, 34' angeordnet. Da die Wandstärke der Durchgangsbegrenzungselemente 6, 6' geringer ist, als die des Auszugskörpers 3, ist die erste Oberfläche 63, 63' beziehungsweise die zweite Oberfläche 64, 64' der Durchgangsbegrenzungselemente 6, 6' in Bezug auf die Vorderseite 30 beziehungsweise die Rückseite 31 jeweils nach innen versetzt. Die Materialaussparungen zur Realisierung der Filmgelenke der Durchgangsbegrenzungselemente 6, 6', sind im Bezug zur Mittelachse des Auszugskörpers 3 alternierend angeordnet, so dass die Durchgangsbegrenzungselemente 6, 6' bei unphysiologischen Auszugsgeschwindigkeiten jeweils alternierend in Richtung der Vorderseite 30 und der Rückseite 31 des Auszugskörpers 3 ausgelenkt werden. Hierdurch wird zum einen eine gleichmäßige Lastverteilung auf den Auszugskörper 3 erreicht, zum anderen kann der durch die Durchgangsbegrenzungselemente 6, 6' erzielte positive Effekt zur Verminderung des Durchgangsquerschnitts des Fluids auf beiden Seiten des Auszugskörpers 3 genutzt werden.

In einer alternativen Ausführungsform können die Durchgangsbegrenzungselemente 6, 6' auch mit der Oberfläche des Auszugskörpers 3, welche in Klapprichtung des jeweiligen Durchgangsbegrenzungselements 6, 6' liegt, fluchten oder über diese hinaus ragen. Ebenfalls können die Durchgangsbegrenzungselemente 6, 6' die gleiche oder eine größere Wandstärke, als der Auszugskörper 3 aufweisen sowie auch außermittig in dem Durchgang 34,34' angeordnet sein. Des Weiteren kann statt der Verbindung mit einem Gelenk 66, 66' auch eine starre Verbindung zwischen Durchgangsbegrenzungselement 6, 6' und Auszugskörper 3 vorgesehen sein.

In Figur 6 ist die orthopädische Vorrichtung 1 aus Figur 5 in einer von der Ausgangsposition abweichenden Position dargestellt. Die Durchgangsbegrenzungselemente 6, 6' sind hier in Richtung der inneren Oberfläche 20 der Aufnahme 2 ausgelenkt. Somit ist der Durchgangsquerschnitt zwischen der inneren Oberfläche 20 der Aufnahme 2 und dem jeweiligen zweiten Ende 62, 62' der Durchgangsbegrenzungselemente 6, 6' im Vergleich zur Ausgangsposition reduziert. Des Weiteren werden aufgrund der Auslenkung der Durchgangsbegrenzungselemente 6, 6' in jedem Durchgang 34, 34' des Auszugskörpers 3 die Durchgänge 34, 34' geöffnet, durch welche bei einer Bewegung des Auszugskörpers 3 nun ebenfalls Fluid strömen kann. So ist die Fluidströmung F des dilatanten Fluids bei einer Bewegung des Auszugskörpers 3 derart aufgeteilt, dass ein erster Teil des dilatanten Fluids durch den Durchgangsquerschnitt und ein zweiter Teil durch den Durchgang 34, 34' des Auszugskörpers 3 strömt, wobei in den beiden Fließwegen der Strömung des dilatanten Fluids im Vergleich zum Fließweg der Strömung durch den Durchgangsquerschnitt in der Ausgangsposition eine höhere Scherrate vorliegt und das dilatante Fluid somit in den beiden geteilten Fließwegen eine erhöhte Viskosität aufweist.

Um das Auslenken der Durchgangsbegrenzungselemente 6, 6' zu begünstigen, ist die Geometrie der Durchgänge 34, 34' und der Durchgangsbegrenzungselemente 6, 6' derart ausgebildet, dass es bei einer Bewegung des Auszugskörpers 3 in Auszugsrichtung A zu einer leicht asymmetrischen Anströmung der Durchgangsbegrenzungselemente 6, 6' kommt. Dies kann beispielsweise durch in Figur 6 nicht dargestellte, jeweils unterschiedlich große Radien an den jeweiligen Kanten der Oberseite 34a der Durchgänge 34, 34' sowie entgegengesetzt an den jeweiligen beiden Kanten des zweiten Endes 62, 62' der Durchgangselemente 6, 6' realisiert werden. Alternativ können natürlich auch andere geometrische Gestaltungen genutzt werden, um die unsymmetrische Anströmung zu erzielen. Weiterhin können die Durchgangsbegrenzungselemente 6, 6' in der Ausgangsposition gegenüber der Auszugsrichtung A unter einem geringen Winkel in Richtung der inneren Oberfläche 20 der Aufnahme 2 geringfügig schräg angeordnet sein.

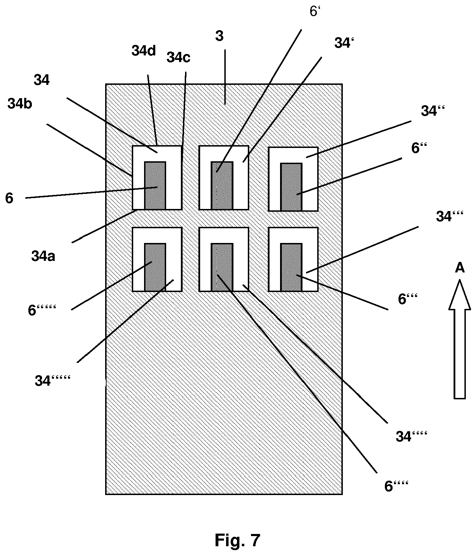

Figur 7 zeigt schematisch eine Vorderansicht des Auszugskörpers 3 aus den Figuren 5 und 6 mit Durchgängen 34, 34', 34", 34'", 34"", 34'"" und darin befindlichen Durchgangsbegrenzungselementen 6, 6', 6", 6'", 6"", 6"": Die Durchgänge 34, 34', 34", 34'", 34"", 34'"" sind in diesem Fall durchgehend vorgesehen und in zwei Reihen zu jeweils drei Stück angeordnet. Die sich in den Durchgängen 34, 34', 34", 34'", 34"", 34'"" befindlichen Durchgangsbegrenzungselemente 6, 6', 6", 6'", 6"", 6"" sind auf der Unterseite 34a der Durchgänge 34, 34', 34", 34'", 34"", 34'"" jeweils mittig vorgesehen und weisen zu den seitlichen Rändern 34b, 34c und der Oberseite 34d der Durchgänge 34, 34', 34", 34'", 34"", 34'"" jeweils einen definierten Abstand auf.

Die in Figur 6 gezeigte gegensätzliche Auslenkung der Durchgangsbegrenzungselemente 6, 6' hat den Vorteil, dass die aufgrund der Durchgangsbegrenzungselemente 6, 6' entstehenden, auf den Auszugskörper 2 wirkenden Kräfte weitest möglich ausgeglichen werden. Entsprechend ist in Figur 7 in Bezug auf ein Durchgangselement 6, 6', 6", 6'", 6"", 6"" die Ausklapprichtung der in der gleichen Reihe benachbarten Durchgangselemente 6, 6', 6", 6'", 6"", 6"" und des sich in der nächsten Reihe direkt darüber beziehungsweise darunter befindenden Durchgangselements 6, 6', 6", 6'", 6"", 6"" entgegengesetzt zur Ausklapprichtung des einen Durchgangselements 6, 6', 6", 6'", 6"", 6"" vorgesehen.

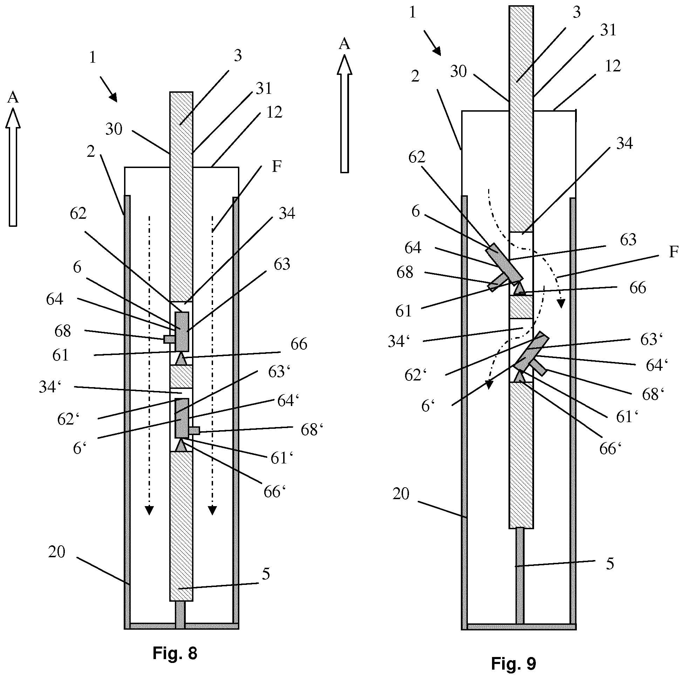

Figur 8 zeigt eine Ausgestaltungsform der orthopädischen Vorrichtung 1 aus Figur 5 in einer Ausgangsposition, wobei die Durchgangsbegrenzungselemente 6, 6' einen senkrecht zur Auszugsrichtung A ausgeprägten, am ersten Ende 61, 61' auf der ersten Oberfläche platzierten Vorsprung 68, 68' aufweisen, welcher in den Durchgangsquerschnitt hineinragt, diesen aber nicht wesentlich beeinflusst. Hierbei ist das Gelenk 66, 66', im vorliegenden Ausführungsbeispiel ein Filmscharnier gemäß Figur 1, derart ausgelegt, dass es bei einem Auszug des Auszugskörpers 3 mit einer physiologischen Auszugsgeschwindigkeit aufgrund der entstehenden Hebelwirkung zu keiner oder einer nur sehr geringen Auslenkung des Durchgangsbegrenzungselements 6, 6' kommt und der Durchgangsquerschnitt in diesem Bereich hierdurch nicht oder nur unwesentlich verändert wird. Weiterhin werden durch den Vorsprung 68, 68' bei diesen Geschwindigkeiten in dem dilatanten Fluid lediglich vernachlässigbar geringe Turbulenzen und Wirbel W erzeugt.

Die orthopädische Vorrichtung 1 aus Figur 8 ist in Figur 9 in einer von der Ausgangsposition abweichenden Position gezeigt. Hierbei sind die Durchgangsbegrenzungselemente 6, 6' zur inneren Oberfläche 20 der Aufnahme 2 hin ausgelenkt. Diese Position nimmt die orthopädische Vorrichtung 1 beispielsweise bei unphysiologischen Körperbewegungen ein. Die Auslenkung der Durchgangsbegrenzungselemente 6, 6' erfolgt hierbei aufgrund der Hebelwirkung der strömungsdynamischen Kräfte auf den jeweiligen Vorsprung 68, 68'. Die Wirkung der Auslenkung der Durchgangsbegrenzungselemente 6, 6' auf die Viskosität des dilatanten Fluids entspricht jener aus den Figuren 2 und 6. Ferner können die Vorsprünge 68, 68' auch zur Begrenzung der Auslenkungsbewegung der Durchgangsbegrenzungselemente 6, 6' dienen.

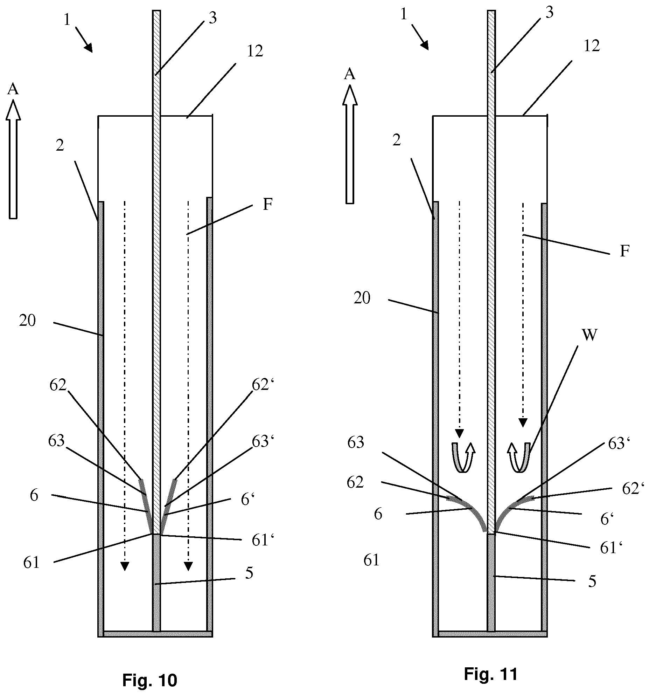

Figur 10 zeigt einer orthopädischen Vorrichtung 1 in Ausgangsposition, wobei der Auszugskörper 3 auf seiner Vorderseite 30 und auf seiner Rückseite 31 jeweils ein ankerförmiges Durchgangsbegrenzungselement 6, 6' aufweist. Das erste Ende 61, 61' der Durchgangsbegrenzungselemente 6, 6' ist jeweils drehfest mit dem Auszugskörper 3 verbunden. In Ausgangsposition beziehungsweise Ruhelage zeigen die Durchgangsbegrenzungselemente 6, 6' in Auszugsrichtung A und weichen von dieser lediglich um einen kleinen Winkel in Richtung der inneren Oberfläche 20 der Aufnahme 2 ab. Somit entspricht die Form einem Anker mit eng anliegenden Flunken. Das zweite Ende 62, 62' eines jeden Durchgangsbegrenzungselements 6, 6' ist dadurch sehr nahe am Auszugskörper 3, wodurch ein relativ großer Durchgangsquerschnitt bereitgestellt wird. Folglich ist der Durchgangsquerschnitt an den Orten der Durchgangsbegrenzungselemente 6, 6' nur unwesentlich geringer, als zwischen der inneren Oberfläche 20 der Aufnahme 2 und der glatten Vorderseite 30 beziehungsweise Rückseite 31 des Auszugskörpers. Bei physiologischen Körperbewegungen bewirken die strömungsbedingt hervorgerufenen Kräfte nur unwesentliche oder keine Veränderungen auf die Geometrie der schirmförmigen Durchgangsbegrenzungselemente 6, 6', folglich ist deren Geometrie bei physiologischen Bewegungen entsprechend jener der Ausgangsposition. Die Ausgestaltung der Durchgangsbegrenzungselemente 6, 6' mit einem spitzen Winkel zur Auszugsrichtung A hat den weiteren Vorteil, dass aufgrund der daraus resultierenden Form beim Rückstellen des Auszugskörpers 3 aus einer ausgezogenen Position in die Ausgangsposition ein geringer Strömungswiderstand ohne oder mit nur sehr geringer Bildung von Turbulenzen und Wirbeln W erzeugt wird. Alternativ kann auch eine beliebige andere Anzahl an Durchgangsbegrenzungselementen 6, 6' an den Auszugskörper 3 angebracht werden.

Figur 11 zeigt die orthopädische Vorrichtung 1 aus Figur 10 in einer von der Ausgangsposition abweichenden Position. Hier sind die Durchgangsbegrenzungselemente 6, 6' entsprechend ihrer Biegesteifigkeit aufgrund der auf die erste Oberfläche 63, 63' wirkenden strömungsdynamischen Kräfte in Richtung der inneren Oberfläche 20 der Aufnahme 2 gebogen. Hierdurch ist das zweite Ende 62, 62' eines jeden Durchgangsbegrenzungselements 6, 6' näher an der inneren Oberfläche 20 der Aufnahme 2, wodurch der Durchgangsquerschnitt im Vergleich zur Ausgangsposition reduziert ist. Die Einnahme dieser Position der orthopädischen Vorrichtung 1 wird typsicherweise durch unphysiologische Körperbewegungen hervorgerufen.

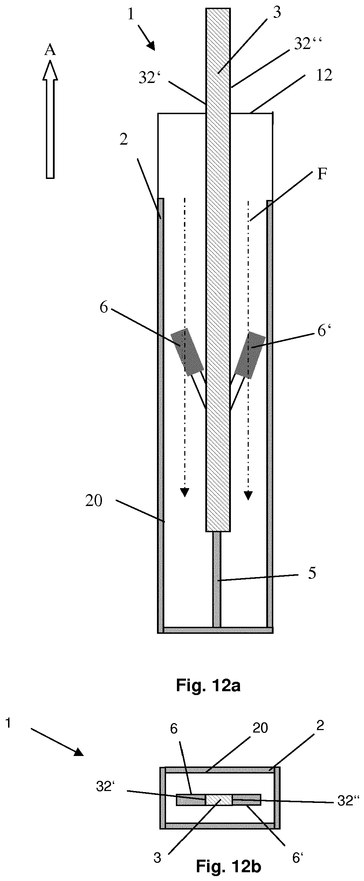

In den Figuren 12a und 12b ist eine Variante einer orthopädischen Vorrichtung 1 mit einem Durchgangsbegrenzungselement 6, 6' in Form eines Torsionskörpers, welcher um die Längsachse des Durchgangsbegrenzungselements 6 tordierbar ist, gezeigt. Die Durchgangsbegrenzungselemente 6, 6' sind an den Seitenflächen 32', 32"des Auszugskörpers 3 angeordnet und befinden sich in einer Ausgangsposition. Die Durchgangselemente weisen eine schmale Seite und eine breite Seite auf. In der Ausgangsposition zeigt die schmale Seite in Auszugsrichtung. Hierdurch ist zwischen dem Durchgangsbegrenzungselement 6, 6' und der inneren Oberfläche 20 der Aufnahme 2 ein größtmöglicher Abstand gegeben.

Die Verdrehung des Durchgangsbegrenzungselements 6, 6' entsteht im vorliegenden Fall durch ein Torsionsmoment, welches beim Ausziehen des Auszugskörpers 3 durch eine in Auszugsrichtung A gesehene asymmetrische Geometrie des Durchgangsbegrenzungselements 6, 6' und einer daraus resultierenden asymmetrischen Anströmung hervorgerufen wird. So weisen die Durchgangsbegrenzungselemente 6, 6' entlang ihrer Längsachse bereits im Ausgangszustand eine leichte Verdrehung auf und sind um eine Krümmungsachse, welche senkrecht zur Längsachse steht, gekrümmt. Es können aber auch andere Möglichkeiten zur Erzeugung des Torsionsmoments auf das Durchgangsbegrenzungselement 6, 6', wie beispielsweise einseitig angebrachte Vorsprünge im Bereich des zweiten Endes 62, 62' des Durchgangsbegrenzungselements 6, 6', genutzt werden.

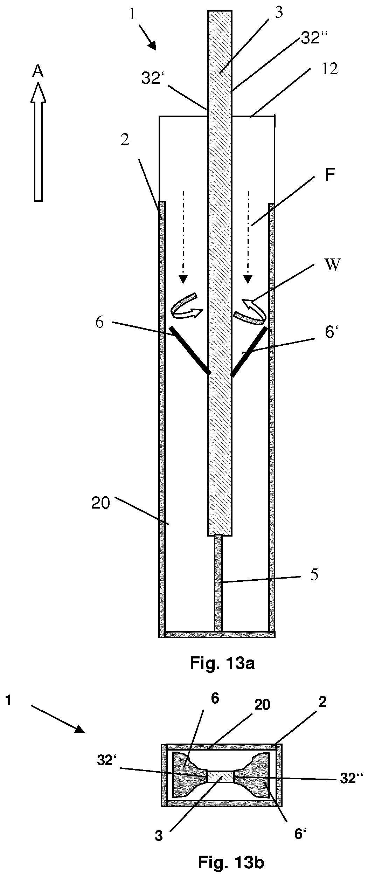

Die Figuren 13a und 13b zeigen die orthopädische Vorrichtung 1 aus den Figuren 12a und 12b in einer von der Ausgangsposition abweichenden Position. Die Durchgangsbegrenzungselemente 6, 6' sind in dieser Position im Vergleich zur Ausgangsposition um einen Verdrehwinkel verdreht, so dass die breite Seite der Durchgangsbegrenzungselemente 6, 6' nun großteils in Auszugsrichtung weist. Hierdurch ist zum einen die projizierte Fläche in Auszugsrichtung A des Durchgangsbegrenzungselements 6, 6' vergrößert, was einen vergrößerten Auszugswiderstand und vermehrte Bildung von Turbulenzen und Wirbel W zur Folge hat. Die projizierte Fläche entsteht, wenn man die Durchgangselemente parallel zur Auszugsrichtung A auf eine Fläche senkrecht zur Auszugsrichtung A projiziert. Zum anderen ist der Abstand zwischen der inneren Oberfläche 20 der Aufnahme 2 und dem Durchgangsbegrenzungselementen 6, 6' reduziert, was einen geringeren Durchgangsquerschnitt generiert. Mit zunehmendem Verdrehwinkel der Durchgangsbegrenzungselemente 6, 6' steigen hierbei der Auszugswiderstand und aufgrund des verkleinerten Durchgangsquerschnitts ebenfalls die Viskosität des dilatanten Fluids.

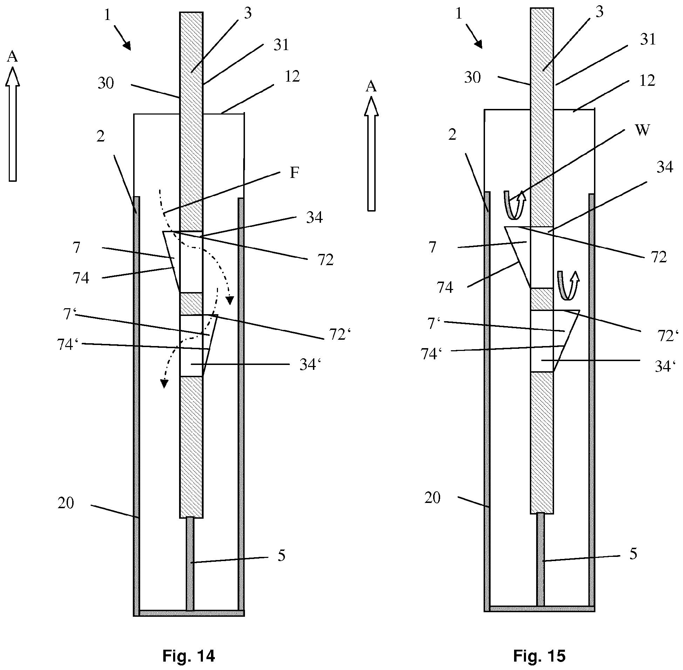

In Figur 14 sind zwei Durchgangsbegrenzungselemente einer dort in einer Ausgangsposition gezeigten Variante einer orthopädischen Vorrichtung 1 in Form von Taschen 7, 7' am Auszugskörper 3 ausgebildet. Diese Taschen 7, 7' umfassen Durchgänge 34, 34' am Auszugskörper 3, welche auf einer Seite jeweils eine schalenartig gestaltete Wand 74, 74'sowie eine Taschenöffnung 72, 72'aufweisen. Durch diese strömt bei Bewegung des Auszugskörpers 3 dilatantes Fluid. Somit stellen die Taschen 7, 7' einen weiteren, diskreten Durchgangsquerschnitt bereit. Bei einem Ausziehen des Auszugskörpers 3 aus der Aufnahme 2 kommt es folglich zur Ausbildung eines ersten Teilstroms des dilatanten Fluids durch den zwischen der der inneren Oberfläche 20 der Aufnahme 2 und der Tasche 7, 7' vorliegenden Durchgangsquerschnitt sowie eines zweiten Teilstroms durch die Taschenöffnung 72, 72'. Die beiden Taschen 7, 7' sind versetzt zu einander angeordnet. Alternativ kann auch eine beliebige andere Anzahl an Taschen 7, 7' am Auszugskörper bereitgestellt werden.

Figur 15 zeigt die orthopädische Vorrichtung 1 aus Figur 14 in einer von der Ausgangsposition abweichenden Position, welche die Vorrichtung 1 bei unphysiologischen Körperbewegungen einnimmt. Hier sind die Taschenöffnungen 72, 72' aufgrund der sich durch die hohen Auszugsgeschwindigkeiten und der daraus resultierenden hohen Scherraten einstellenden hohen Viskosität des dilatanten Fluids im Bereich der Taschenöffnungen 72, 72' durch die Scherverfestigung blockiert, wodurch der gesamte Strom des dilatanten Fluids nun durch den Durchgangsquerschnitt zwischen der inneren Oberfläche 20 der Aufnahme 2 und der Tasche 7, 7' geleitet wird. Hierdurch kommt es dort zu einer starken Erhöhung der Scherrate. Zusätzlich bewirkt die Verblockung der Taschenöffnung 72, 72' eine Zunahme an Turbulenzen und Wirbeln W, welche ebenfalls zu einer Viskositätssteigerung des dilatanten Fluids beitragen.

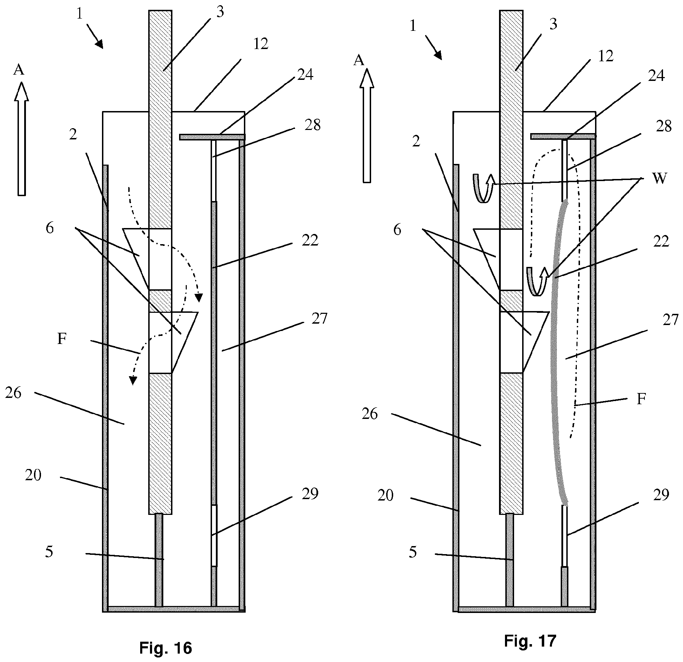

In Figur 16 ist die orthopädische Vorrichtung 1 aus Figur 14 in einer Ausgangsposition gezeigt, wobei die Aufnahme eine Membrane 22 aufweist, welche das Innere der Aufnahme in eine erste Kammer 26 zum Aufnehmen des Auszugskörpers 3 und eine zweite Kammer 27 teilt. Alternativ kann auch jede andere Ausführungsform der Durchgangsbegrenzungselemente 6 des Auszugskörpers 3 mit der die Membrane 22 aufweisende Aufnahme 2 kombiniert werden. Die Membrane 22 weist eine Einlassöffnung 28 im Bereich eines zweiten Endes der Aufnahme 2 zum Aufnehmen von dilatantem Fluid in die zweite Kammer 27, und im Bereich eines ersten Endes der Aufnahme 2 eine Auslassöffnung 29 zum Auslassen von dilatantem Fluid aus der zweiten Kammer 27 auf. Der Einlassöffnung 28 ist ein Abstreifer 24 vorgelagert, um das dilatante Fluid von der ersten Kammer 26 durch die Einlassöffnung 28 in die zweite Kammer 27 zu führen. Die Einlassöffnung 28 ist bei dieser Variante größer ausgebildet, als die Auslassöffnung 29. Alternativ können die Größenverhältnisse aber je nach Auslegung der orthopädische Vorrichtung 1 anders ausgestaltet sein.

Die zweite Kammer 27 verläuft parallel zum Auszugskörper 3. Aufgrund der elastischen Eigenschaften der Membrane 22 dehnt sich diese bei Einströmen des dilatanten Fluids aus der ersten Kammer 26 in die zweite Kammer 26 senkrecht zur Auszugsrichtung A zum Auszugskörper 3 hin aus. So wird mit zunehmend zurückgelegtem Weg des Auszugskörpers 3 in Auszugsrichtung A durch die zunehmend in die zweite Kammer 27 geleitete Menge an dilatantem Fluid die Membrane 22 zunehmend in Richtung Auszugskörper verschoben, was den Durchgangsquerschnitt zusätzlich reduziert und somit eine noch stärkere Rückhaltekraft bereitgestellt werden kann.

Figur 17 zeigt die orthopädische Vorrichtung aus Figur 16 in einer Position, bei der die Membrane 22 in Richtung des Auszugskörpers 3 ausgedehnt ist. In dieser Position ist der Durchgangsquerschnitt zwischen dem Durchgangsbegrenzungselement 6 und der Membrane 22 in der ersten Kammer 26 durch die Dehnung der Membrane 22 senkrecht zur Auszugsrichtung A zum Auszugskörper 3 hin zusätzlich reduziert.

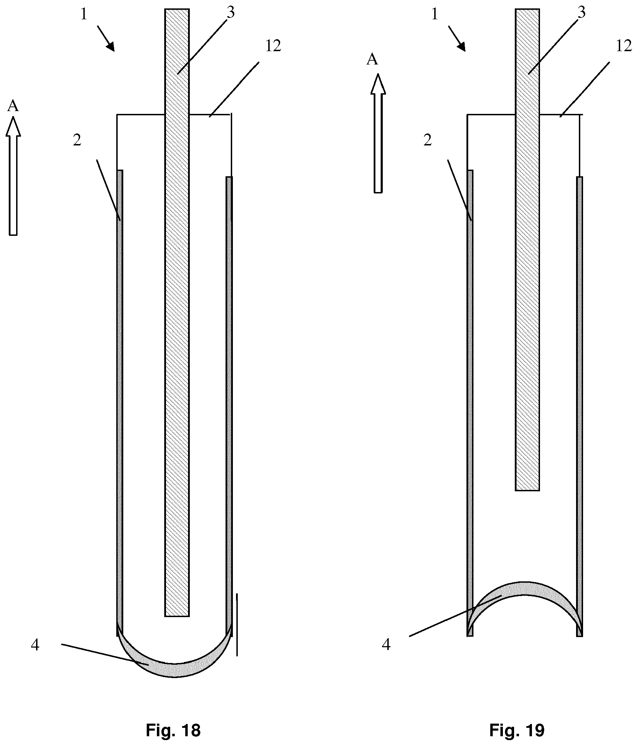

In Figur 18 ist eine orthopädische Vorrichtung 1 gezeigt, deren Aufnahme 2 eine aus Gummi bestehende, elastische Ausgleichsmembran 4 aufweist, welche an der unteren Seite der Aufnahme 2 befestigt ist. Die orthopädische Vorrichtung 1 befindet sich in einer Ausgangsposition, in der der Auszugskörper 3 bezüglich des Bewegungsumfangs der orthopädischen Vorrichtung 1 eingefahren ist. Die Ausgleichsmembran 4 befindet sich in Bezug auf das Innere der Aufnahme 2 in einer nach außen gewölbten Gestalt. Hierbei ist das komplette Volumen innerhalb der Aufnahme 2 bis zur Dichtung 12 mit dilatantem Fluid gefüllt.

Figur 19 zeigt die orthopädische Vorrichtung 1 aus Figur 18 in einer von der Ausgangsposition abweichenden Position. Der Auszugkörpers 3 ist im Vergleich zur Ausgangsposition um einen bestimmten Betrag in Auszugsrichtung A ausgezogen worden, wodurch das Volumen im Inneren der Aufnahme 2 entsprechend reduziert wurde. Die Ausgleichsmembran 4 gleicht den hierdurch entstandenen Unterdruck aus, indem sie sich entsprechend des verdrängten Volumens innerhalb der Aufnahme 2 in Auszugsrichtung A beult. Im vorliegenden Fall weist die Ausgleichsmembran 4 in Bezug auf das Innere de Aufnahme 2 eine nach innen gewölbte Gestalt auf. Die Ausgleichsmembran 4 kann auch jede andere Form, welche ihr durch die Lage des Auszugskörpers 3 relativ zur Aufnahme 2 vorgegeben wird, annehmen.

Durch den Ausgleich des Volumens innerhalb der Aufnahme 2 kann beim Ausziehen des Auszugskörpers 3 ein Vakuum- Effekt, welcher sich nachteilig auf die Viskosität des dilatanten Fluids auswirken und überdies eine zusätzliche Rückhaltekraft auf den Auszugskörper 3 hervorrufen könnte, vermieden werden. Somit kommt es durch die Ausgleichsmembran 4 zu einem Druckausgleich innerhalb der Aufnahme 2. Dadurch kann insbesondere im Bereich physiologischer Bewegungen im Vergleich zu einer starren Aufnahme 2 eine nochmals erhöhte Leichtgängigkeit der orthopädischen Vorrichtung 1 bereitgestellt werden. Die Ausgleichsmembran 4 kann alternativ auch aus einem anderen flexiblen Material, wie beispielsweise Latex oder einem thermoplastischen Polyurethan hergestellt sein.

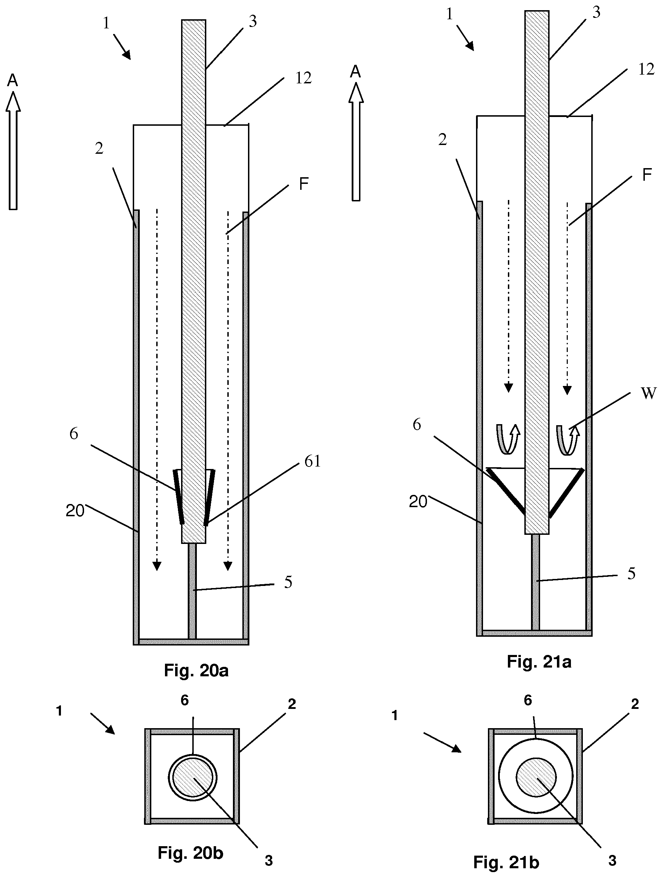

Figur 20a zeigt schematisch eine Vorderansicht im Querschnitt durch eine orthopädische Vorrichtung 1 mit einem Durchgangsbegrenzungselement 6 in Form eines Schirms in einer Ausgangsposition, derart, dass der Auszugskörper 3 von dem Durchgangsbegrenzungselement 6 schirmförmig umgeben ist. In der Ausgangsposition bilden das schirmförmige Durchgangsbegrenzungselement 6 und der Auszugskörper 3 einen geschlossenen Schirm. Dabei bildet das erste, den Auszugskörper umlaufende Ende 61 des Durchgangsbegrenzungselements 6 eine drehfeste Verbindung mit dem Auszugskörper 3. Das schirmförmige Durchgangsbegrenzungselement 6 ist aus Kautschuk bereitgestellt.

Bei physiologischen Auszugsgeschwindigkeiten erfährt das schirmförmige Durchgangsbegrenzungselement 6 keine oder nur eine geringe Auslenkung, so dass die Fluidströmung F relativ zum Auszugskörper lediglich eine geringe Beeinflussung durch das schirmförmige Durchgangsbegrenzungselement 6 erfährt.

In Figur 20b ist schematisch eine Draufsicht in einer Schnittdarstellung der orthopädischen Vorrichtung 1 aus Figur 20a gezeigt. Durch das nahe Anliegen des schirmförmigen Durchgangsbegrenzungselements 6 an dem Auszugskörper 3 wird ein großer Durchgangsquerschnitt bereitgestellt.

Figur 21a zeigt schematisch eine Vorderansicht im Querschnitt durch die orthopädische Vorrichtung 1 gemäß Figur 20a in einer von der Ausgangsposition abweichenden Position. Das schirmförmige Durchgangsbegrenzungselement 6 ist im Vergleich zur Ausgangsposition in Richtung der Aufnahme 2 aufgespannt, wodurch der Durchgangsquerschnitt zwischen der Aufnahme 2 und dem schirmförmigen Durchgangsbegrenzungselement 6 reduziert ist.

In Figur 21b ist schematisch eine Draufsicht in einer Schnittdarstellung der orthopädischen Vorrichtung 1 aus Figur 21a gezeigt. Durch das aufgespannte schirmförmige Durchgangsbegrenzungselement 6 ist der Durchgangsquerschnitt im Vergleich zur Ausgangsposition reduziert bereitgestellt. Die aufgespannte Position nimmt das schirmförmige Durchgangsbegrenzungselement 6 bei unphysiologischen Auszugsgeschwindigkeiten ein und bewirkt hierdurch eine Zunahme der Viskosität des dilatanten Fluids zwischen dem schirmförmigen Durchgangsbegrenzungselement 6 und der Aufnahme 2 sowie eine Zunahme an Turbulenzen und Wirbel W im dilatanten Fluid.

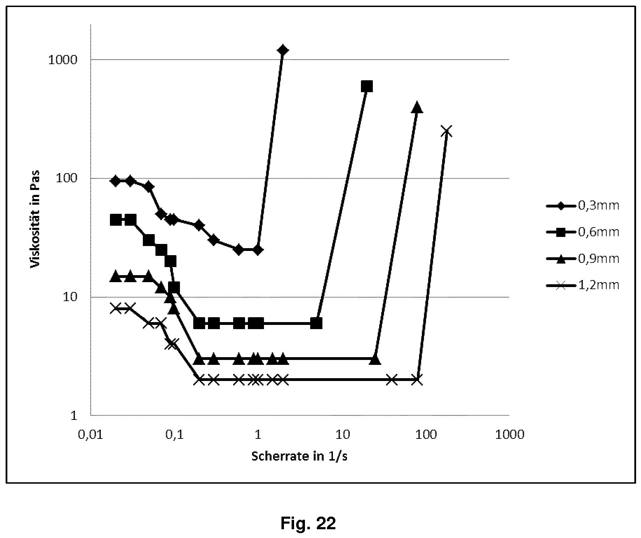

Figur 22 zeigt schematisch den Viskositätsverlauf eines hier nicht näher beschriebenen dilatanten Fluids in Bezug auf die Scherrate für verschiedene Durchgansquerschnitte, wobei die Viskosität in Pas logarithmisch auf der Ordinatenachse und die Scherrate in s⁻¹ auf der Abszissenachse aufgetragen sind. Die Durchgangsquerschnitte unterscheiden sich hierbei einzig in den Abständen zwischen dem Auszugskörper und der inneren Oberfläche der Aufnahme, welche in der Legende auf der rechten Seite der Figur 22 aufgelistet sind. Alle vier Kurven zeigen das typische verhalten dilatanter Fluide. Nach einer anfänglichen Scherverdünnung aufgrund von zunehmender Homogenisierung des Fluids infolge der zunehmenden Partikelbewegung kommt es ab einer kritischen Scherrate zu einem sprunghaften Anstieg der Viskosität, zum sogenannten Dilatanzsprung. Mit anderen Worten kommt es ab dem Dilatanzsprung im dilatanten Fluid zu einer schlagartigen Scherverfestigung.

Aus Figur 22 ist weiterhin ersichtlich, dass mit abnehmendem Durchgangsquerschnitt zum einen die Viskosität bei konstanter Scherrate ansteigt, zum anderen der Dilatanzsprung bereits bei einer geringeren Scherrate einsetzt. Somit kann durch gezielte konstruktive Einstellung des Durchgangsquerschnitts einer orthopädischen Vorrichtung 1 sowohl die sich einstellende Viskosität, als auch die Lage des Dilatanzsprungs auf den jeweiligen Anwendungsfall angepasst werden.

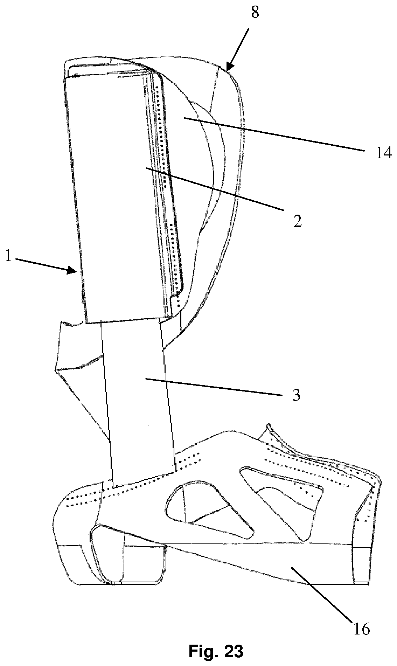

Figur 23 zeigt eine Sprunggelenksorthese 8, welche eine orthopädische Vorrichtung 1 aufweist. Die Orthese ist um ein hier nicht gezeigtes Sprunggelenk an zwei hier nicht gezeigten Körperbereichen angeordnet, derart, dass sich der Ort des Sprunggelenks zwischen einem ersten Orthesenbereich 14 und einem zweiten Orthesenbereich 16 befindet. Der erste Orthesenbereich 14 und der zweite Orthesenbereich 16 sind hierbei über die orthopädische Vorrichtung 1 verbunden, wobei die Aufnahme 2 an dem ersten Orthesenbereich 14, und der Auszugskörper 3 an dem zweiten Orthesenbereich 16 angebracht ist. Bei einer Supinationsbewegung des Sprunggelenks kommt es entsprechend des Winkels der Supination zu einem Auszug des Auszugskörpers 3 aus der Aufnahme in der Größenordnung des aus dem Winkel der Supination und dem Abstand zwischen Auszugskörper 3 und Gelenkmittelpunkt resultierenden Bogenmaßes. Im Bereich physiologischer Bewegungen ist der Auszug des Auszugskörpers 3 nicht oder nur geringfügig gehemmt. Liegt die Supinationsgeschwindigkeit im unphysiologischen Bereich, so kommt es aufgrund der daraus resultierenden starken Rückhaltekraft der orthopädischen Vorrichtung 1 zu einer Bewegungshemmung der beiden Orthesenbereiche 14, 16 und somit der beiden Körperbereiche zueinander.

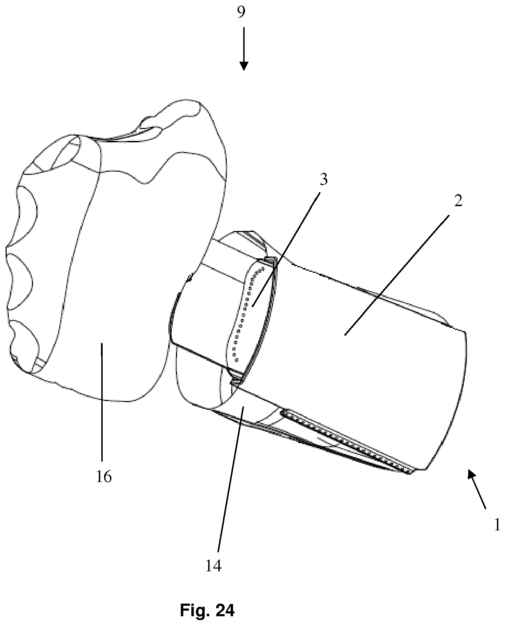

Figur 24 ist eine Handgelenksorthese 9 zu entnehmen. Diese weist einen ersten Orthesenbereich 14 distal und einem zweiten Orthesenbereich 16 proximal eines hier nicht gezeigten Handgelenks auf. Der erste Orthesenbereich 14 und der zweite Orthesenbereich 16 sind über die orthopädische Vorrichtung 1 miteinander verbunden, derart, dass der Auszugskörper 3 an dem ersten Orthesenbereich 14 und die Aufnahme 2 an dem zweiten Orthesenbereich 16 befestigt ist. Bei einer Dorsalextension des Handgelenks kommt es entsprechend des von dem Dorsalextensionswinkel und dem Abstand zwischen der Handgelenksdrehachse und der Vorrichtung abhängigen Bogenmaßes zu einem Auszug des Auszugskörpers 3 aus der Aufnahme 2. Im Bereich physiologischer Bewegungen ist der Auszug des Auszugskörpers 3 nicht oder nur geringfügig gehemmt. Liegt die Dorsalextensionsgeschwindigkeit im unphysiologischen Bereich, so kommt es aufgrund der daraus resultierenden starken Rückhaltekraft der orthopädischen Vorrichtung 1 zu einer Bewegungshemmung der beiden Orthesenbereiche 14, 16 und somit der beiden Körperbereiche zueinander.

### Bezugszeichenliste

- 1: Orthopädische Vorrichtung

- 2: Aufnahme
- 20: Innere Oberfläche
- 22: Membrane
- 24: Abstreifer
- 26: Erste Kammer
- 27: Zweite Kammer
- 28: Einlassöffnung
- 29: Auslassöffnung

- 3: Auszugskörper
- 30: Vorderseite
- 31: Rückseite
- 32', 32": Seitenflächen
- 34, 34', 34", 34'", 34"", 34'"": Durchgang
- 34a: Unterseite
- 34b,34c: Seitliche Ränder
- 34d: Oberseite
- 36: Aussparung
- 37: Innenseite

- 4: Ausgleichsmembran

- 5: Rückstellung

- 6, 6', 6", 6'", 6"", 6"": Durchgangsbegrenzungselement
- 61, 61': Erstes Ende
- 62, 62': Zweites Ende
- 63, 63': Erste Oberfläche
- 64, 64': Zweite Oberfläche
- 66, 66': Gelenk
- 68, 68': Vorsprung

- 7, 7': Tasche
- 72, 72': Taschenöffnung
- 74, 74': Schalenartige Wand

- 8: Sprunggelenksorthese

- 9: Handgelenksorthese

- 12: Dichtung

- 14: Erster Orthesenbereich

- 16: Zweiter Orthesenbereich

- A: Auszugsrichtung
- F: Fluidströmung relativ zum Auszugskörper
- W: Turbulenzen und Wirbel

## Patentansprüche

1. Orthopädische Vorrichtung (1) zur Begrenzung der Bewegung eines zwischen einem ersten und einem zweiten Körperbereich angeordneten Gelenks, umfassend zumindest eine an dem ersten Körperbereich fixierbare Aufnahme (2) und einen an dem zweiten Körperbereich fixierbaren und relativ zu der Aufnahme (2) bewegbaren Auszugskörper (3), wobei senkrecht zu einer Auszugsrichtung zwischen der Aufnahme (2) und dem Auszugskörper (3) ein mit einem dilatanten Fluid gefüllter Durchgangsquerschnitt bereitgestellt ist,
**dadurch gekennzeichnet, dass**
mindestens ein Durchgangsbegrenzungselement (6) zur Veränderung des Durchgangsquerschnitts vorgesehen ist.

2. Orthopädische Vorrichtung (1) gemäß Anspruch 1, **dadurch gekennzeichnet, dass** das mindestens eine Durchgangsbegrenzungselement (6) plattenförmig, scheibenförmig und/oder stabförmig ist.

3. Orthopädische Vorrichtung (1) gemäß Anspruch 1 oder 2, **dadurch gekennzeichnet, dass** das mindestens eine Durchgangsbegrenzungselement (6) zum Auslenken aus einer Ausgangsposition elastisch ist und an einem ersten Ende fest, bevorzugt drehfest, mit dem Auszugskörper oder der Aufnahme verbunden ist.

4. Orthopädische Vorrichtung (1) gemäß Anspruch 1 oder 2, **dadurch gekennzeichnet, dass** das mindestens eine Durchgangsbegrenzungselement (6) zum Auslenken aus einer Ausgangsposition an einem ersten Ende über ein Gelenk (66), bevorzugt ein Scharnier und besonders bevorzugt ein Filmscharnier, mit dem Auszugskörper (3) oder der Aufnahme (2) verbunden ist.

5. Orthopädische Vorrichtung (1) gemäß einem der vorhergehenden Ansprüche, **dadurch gekennzeichnet, dass** das mindestens eine Durchgangsbegrenzungselement (6) in einer Ausgangsposition zu dem Auszugskörper (3) oder der Aufnahme (2) in einem spitzen Winkel angeordnet ist.

6. Orthopädische Vorrichtung (1) gemäß einem der vorhergehenden Ansprüche, **dadurch gekennzeichnet, dass** das mindestens eine Durchgangsbegrenzungselement (6) den Auszugskörper (3) schirmförmig umgibt.

7. Orthopädische Vorrichtung (1) gemäß einem der vorhergehenden Ansprüche, **dadurch gekennzeichnet, dass** der Auszugskörper (3) und/oder die Aufnahme (2) mindestens eine Aussparung (36) aufweist, in welcher mindestens ein Durchgangsbegrenzungselement (6) in einer Ausgangsposition versenkt ist.

8. Orthopädische Vorrichtung (1) gemäß einem der vorhergehenden Ansprüche, **dadurch gekennzeichnet, dass** der Auszugskörper (3) mindestens einen Durchgang (34) zum Bereitstellen eines zusätzlichen Fließweges für das dilatante Fluid aufweist, in welcher mindestens ein Durchgangsbegrenzungselement (6) in einer Ausgangsposition versenkt ist.

9. Orthopädische Vorrichtung (1) gemäß Anspruch 7 oder 8, **dadurch gekennzeichnet, dass** das mindestens eine Durchgangsbegrenzungselement (6) mindestens einen Vorsprung (68) zur Aktivierung einer Auslenkung des mindestens einen Durchgangsbegrenzungselements (6) aufweist, welcher in den Durchgangsquerschnitt hineinragt.

10. Orthopädische Vorrichtung (1) gemäß Anspruch 1, **dadurch gekennzeichnet, dass** das mindestens eine Durchgangsbegrenzungselement (6) eine Tasche (7) aufweist, um einen Teil des dilatanten Fluids in einen Durchgang des Auszugskörpers umzulenken, wobei eine Taschenöffnung (72) in eine Bewegungsrichtung des Auszugskörpers (3), bevorzugt die Auszugsrichtung (A) des Auszugskörpers (3) zeigt.

11. Orthopädische Vorrichtung (1) gemäß einem der vorhergehenden Ansprüche, **dadurch gekennzeichnet, dass** zwischen der Aufnahme (2) und dem Auszugskörper (3) eine elastische Membrane (22) parallel zur Auszugsrichtung bereitgestellt ist, welche eine Einlassöffnung (28) im Bereich eines zweiten Endes der Aufnahme zum Aufnehmen von dilatantem Fluid und im Bereich eines ersten Endes der Aufnahme eine Auslassöffnung (29) zum Auslassen von dilatantem Fluid aufweist, wobei die Membrane (22) das Innere der 3 Aufnahme (2) in eine erste Kammer (26) zum Aufnehmen des Auszugskörpers (3) und eine zweite Kammer (27) teilt, wobei der Einlassöffnung (28) ein Abstreifer (24) vorgelagert ist, um das dilatante Fluid von der ersten Kammer (26) durch die Einlassöffnung (28) in die zweite Kammer (27) zuführen.

12. Orthopädische Vorrichtung (1) gemäß einem der vorhergehenden Ansprüche, **dadurch gekennzeichnet, dass** die Aufnahme (2) elastisch ist.

13. Orthopädische Vorrichtung (1) gemäß einem der vorhergehenden Ansprüche, **dadurch gekennzeichnet, dass** die Aufnahme (2) eine Flechtstruktur, bevorzugt einen Ziehstrumpf umfasst, welche einen Aufnahmekörper umschließt.

14. Orthopädische Vorrichtung (1) gemäß einem der vorhergehenden Ansprüche, **dadurch gekennzeichnet, dass** zwischen der Aufnahme (2) und dem Auszugskörper (3) ein Ziehstrumpf zum abschließen der mit dilatantem Fluid gefüllten Aufnahme (2) angeordnet ist, wobei ein erstes Ende des Ziehstrumpfes an einem oberen Abschnitt des Auszugskörpers (3) umfänglich befestigt ist, und ein zweites Ende des Ziehstrumpfes an einem Öffnungsrand der Aufnahme (2) umlaufend befestigt ist.

15. Orthopädische Vorrichtung (1) gemäß einem der vorhergehenden Ansprüche, **dadurch gekennzeichnet, dass** das mindestens eine Durchgangsbegrenzungselement (6) ein Torsionskörper ist, welcher um die Längsachse des Durchgangsbegrenzungselements (6) von einer Ausgangsposition in eine Begrenzungsposition und von der Begrenzungsposition in die Ausgangsposition verdrehbar ist, wobei in einer Ausgangsposition eine schmale Seite des Durchgangsbegrenzungselements (6) in eine Auszugsrichtung (A) zeigt und in einer Begrenzungsposition eine breite Seite des Durchgangsbegrenzungselements (6) in die Auszugsrichtung (A) zeigt.

16. Orthopädische Vorrichtung (1) gemäß einem der vorhergehenden Ansprüche, **dadurch gekennzeichnet, dass** die Aufnahme (2) in zumindest einem Bereich eine elastische Ausgleichsmembran (4) zum Ausgleichen einer nach einer Positionsänderung des Auszugskörpers (3) hervorgerufenen Volumenänderung aufweist.

17. Orthopädische Vorrichtung (1) gemäß einem der vorhergehenden Ansprüche, **dadurch gekennzeichnet, dass** der Abstand zwischen dem Auszugskörper (3) und der Aufnahme (2) senkrecht zur Auszugskörperrichtung in der Ausgangsposition zwischen 0,01 und 1000 mm, bevorzugt 0,1 bis 50 mm, besonders bevorzugt 0,1 bis 15 mm, und ganz besonders bevorzugt 0,1 bis 5 mm beträgt.

18. Orthopädische Vorrichtung (1) gemäß einem der vorhergehenden Ansprüche, **dadurch gekennzeichnet, dass** das mindestsens eine Durchgangsbegrenzungselement (6) Kautschuk oder Kunststoff, bevorzugt Silicon oder thermoplastischem Kunststoff, besonders bevorzugt Polypropylen, Polyethylen und/oder Polyurethan aufweist.

19. Orthopädische Vorrichtung (1) gemäß einem der vorhergehenden Ansprüche, **dadurch gekennzeichnet, dass** das dilatante Fluid eine Dispersion aus Ethylenglykol oder Silikon-Öl und Siliciumdioxid, bevorzugt Silica Gel mit einer Teilchengröße von 2 bis 1000 nm, Oberflächen von 30 bis 250 m²/g, einen Feststoffanteil von 5 bis 80 Gew.-% und Stabilisatoren umfasst.

## Claims

1. An orthopedic device (1) for limiting the movement of a joint disposed between a first body portion and a second body portion, comprising at least one reception (2) fixable to the first body portion, and a pull-out body (3) fixable to the second body portion and movable relative to the reception (2), wherein perpendicular to a pull out direction between the reception (2) and the pull-out body (3) a passage cross section filled with a shear thickening fluid is provided,
**characterized in that**
at least one passage limiting element (6) for changing the passage cross section is provided.

2. The orthopedic device (1) according to claim 1, **characterized in that** the at least one passage limiting element (6) is plate shaped, disc shaped, and/or rod shaped.

3. The orthopedic device (1) according to claim 1 or 2, **characterized in that** the at least one passage limiting element (6) is flexible and on a first end firmly, preferably in a torque proof manner, connected to the pull-out body or the reception, in order to deflect from a starting position.

4. The orthopedic device (1) according to claim 1 or 2, **characterized in that** the at least one passage limiting element (6) on a first end is connected to the pull-out body (3) or the reception (2) by means of a joint (66), preferably a hinge, and more preferably a film hinge, in order to deflect from a starting position.

5. The orthopedic device (1) according to one of the preceding claims, **characterized in that** the at least one passage limiting element (6) is disposed in an acute angle to the pull-out body (3) or the reception (2) in a starting position.

6. The orthopedic device (1) according to one of the preceding claims, **characterized in that** the pull-out body (3) is surrounded by at least one umbrella shaped passage limiting element (6).

7. The orthopedic device (1) according to one of the preceding claims, **characterized in that** at least one of the pull-out body (3) and the reception (2) have at least one recess (36) in which at least one passage limiting element (6) is recessed in a starting position.

8. The orthopedic device (1) according to one of the preceding claims, **characterized in that** the pull-out body (3) has at least one passage (34) for providing an additional flow path for the shear thickening fluid, in which at least one passage limiting element (6) is recessed in a starting position.

9. The orthopedic device (1) according to claim 7 or 8, **characterized in that** the at least one passage limiting element (6) has at least one protrusion (68) protruding into the passage cross-section for the activation of a deflection of the at least one passage limiting element (6).

10. The orthopedic device (1) according to claim 1, **characterized in that** the at least one passage limiting element (6) has a pouch (7) in order to redirect a part of the shear thickening fluid into a passage of the pull-out body, wherein an opening (72) of the pouch points in the direction of the movement of the pull-out body (3), preferably in the pull-out direction (A) of the pull-out body (3).

11. The orthopedic device (1) according to one of the preceding claims, **characterized in that** between the reception (2) and the pull-out body (3) a flexible membrane (22) is provided in parallel to the pull-out direction, wherein the membrane has an inlet opening (28) in the area of a second end of the reception for receiving the shear thickening fluid, and an outlet opening (29) in the area of a first end of the reception for letting out the shear thickening fluid, wherein the membrane (22) divides the inside of the reception (2) into a first chamber (26) for receiving the pull-out body (3) and a second chamber (27), wherein a stripper (24) is disposed in front of the inlet opening (28) in order to lead the shear thickening fluid from the first chamber (26) through the inlet opening (28) into the second chamber (27).

12. The orthopedic device (1) according to one of the preceding claims, **characterized in that** the reception (2) is flexible.

13. The orthopedic device (1) according to one of the preceding claims, **characterized in that** the reception (2) comprises a weaving structure, preferably a pulling grip, which encloses a reception body.

14. The orthopedic device (1) according to one of the preceding claims, **characterized in that** a pulling grip for closing the reception (2) filled with the shear thickening fluid is disposed between the reception (2) and the pull-out body (3), wherein a first end of the pulling grip is circumferentially attached to an upper section of the pull-out body (3), and a second end of the pulling grip is circumferentially attached to an opening edge of the reception (2).

15. The orthopedic device (1) according to one of the preceding claims, **characterized in that** the at least one passage limiting element (6) is a torsion body which is twistable around the longitudinal axis of the at least one passage limiting element (6) from a starting position to a limiting position and from the limiting position to the starting position, wherein in the starting position a narrow side of the at least passage limiting element (6) points into a pull-out direction (A) and in a limiting position a broad side of the at least one passage limiting element (6) points into the pull-out direction (A).

16. The orthopedic device (1) according to one of the preceding claims, **characterized in that** the reception (2) has an elastic balancing membrane (4) in at least one area, which can balance a change of volume within the reception (2) caused by a movement of the pull-out body (3).

17. The orthopedic device (1) according to one of the preceding claims, **characterized in that** the distance between the pull-out body (3) and the reception (2) perpendicular to the pull-out direction in the starting position amounts between 0.01 to 1000 mm, preferably 0.1 to 50 mm, and more preferably 0.1 to 15 mm and even more preferably 0.1 to 5 mm.

18. The orthopedic device (1) according to one of the preceding claims, **characterized in that** the at least one passage limiting element (6) comprises natural rubber or plastic, preferably silicon or thermoplastic resin, more preferably polypropylene, polyethylene and/or polyurethane.

19. The orthopedic device (1) according to one of the preceding claims, **characterized in that** the shear thickening fluid comprises a dispersion of ethylenglycol or silicone oil and siliciumdioxid, preferably silica gel with a particle size of 2 to 1000 nm, surfaces of 30 to 250 m²/g, and a solid content of 5 to 80 weight-% and stabilizers.

## Revendications

1. Dispositif orthopédique (1) destiné à limiter le mouvement d'une articulation située entre une première partie du corps et une deuxième partie du corps, comprenant au moins un logement (2) qui peut être fixé à la première partie du corps et un corps d'allongement (3) qui peut être fixé à la deuxième partie du corps et qui peut être mobile par rapport au logement (2), une section transversale de passage remplie d'un fluide dilatant étant prévue entre le logement (2) et le corps d'allongement (3) perpendiculairement à une direction d'allongement,
**caractérisé en ce qu'**il est prévu au moins un élément limiteur de passage (6) destiné à modifier la section transversale de passage.

2. Dispositif orthopédique (1) selon la revendication 1, **caractérisé en ce que** l'au moins un élément limiteur de passage (6) est en forme de plaque, de disque et/ou de barre.

3. Dispositif orthopédique (1) selon la revendication 1 ou 2, **caractérisé en ce que**, afin de dévier d'une position initiale, l'au moins un élément limiteur de passage (6) est élastique et est assemblé par une première extrémité de manière fixe, de préférence de manière fixe en rotation, au corps d'allongement ou au logement.

4. Dispositif orthopédique (1) selon la revendication 1 ou 2, **caractérisé en ce que**, afin de dévier d'une position initiale, l'au moins un élément limiteur de passage (6) est assemblé par une première extrémité par l'intermédiaire d'une articulation (66), de préférence une charnière et de manière particulièrement préférée une charnière à film, au corps d'allongement (3) ou au logement (2).

5. Dispositif orthopédique (1) selon l'une quelconque des revendications précédentes, **caractérisé en ce que**, dans une position initiale, l'au moins un élément limiteur de passage (6) est agencé suivant un angle aigu par rapport au corps d'allongement (3) ou au logement (2).

6. Dispositif orthopédique (1) selon l'une quelconque des revendications précédentes, **caractérisé en ce que** l'au moins un élément limiteur de passage (6) entoure en forme d'écran le corps d'allongement (3).

7. Dispositif orthopédique (1) selon l'une quelconque des revendications précédentes, **caractérisé en ce que** le corps d'allongement (3) et/ou le logement (2) comporte au moins un évidement (36) dans lequel au moins un élément limiteur de passage (6) est enfoncé dans une position initiale.

8. Dispositif orthopédique (1) selon l'une quelconque des revendications précédentes, **caractérisé en ce que** le corps d'allongement (3) comporte au moins un passage (34) destiné à fournir une voie d'écoulement supplémentaire pour le fluide dilatant, passage dans lequel au moins un élément limiteur de passage (6) est enfoncé dans une position initiale.

9. Dispositif orthopédique (1) selon la revendication 7 ou 8, **caractérisé en ce que** l'au moins un élément limiteur de passage (6) comporte au moins une partie en saillie (68) destinée à activer une déviation de l'au moins un élément limiteur de passage (6), partie en saillie qui pénètre dans la section transversale de passage.

10. Dispositif orthopédique (1) selon la revendication 1, **caractérisé en ce que** l'au moins un élément limiteur de passage (6) comporte une poche (7) pour dévier une partie du fluide dilatant dans un passage du corps d'allongement, une ouverture de poche (72) pointant alors dans une direction de mouvement du corps d'allongement (3), de préférence la direction d'allongement (A) du corps d'allongement (3).

11. Dispositif orthopédique (1) selon l'une quelconque des revendications précédentes, **caractérisé en ce qu'**une membrane élastique (22) est prévue parallèlement à la direction d'allongement, entre le logement (2) et le corps d'allongement (3), laquelle membrane élastique comporte une ouverture d'entrée (28) dans la zone d'une deuxième extrémité du logement afin de recevoir du fluide dilatant et une ouverture de sortie (29) dans la zone d'une première extrémité du logement afin d'évacuer du fluide dilatant, laquelle membrane (22) sépare l'intérieur du logement (2) en une première chambre (26) destinée à recevoir le corps d'allongement (3) et une deuxième chambre (27), une racle (24) étant placée en amont de l'ouverture d'entrée (28) pour conduire le fluide dilatant de la première chambre (26) à la deuxième chambre (27) via l'ouverture d'entrée (28).

12. Dispositif orthopédique (1) selon l'une quelconque des revendications précédentes, **caractérisé en ce que** le logement (2) est élastique.

13. Dispositif orthopédique (1) selon l'une quelconque des revendications précédentes, **caractérisé en ce que** le logement (2) comprend une structure tressée, de préférence une chaussette, qui entoure un corps de logement.

14. Dispositif orthopédique (1) selon l'une quelconque des revendications précédentes, **caractérisé en ce qu'**une chaussette destinée à fermer le logement (2) rempli de fluide dilatant est agencée entre le logement (2) et le corps d'allongement (3), une première extrémité de la chaussette étant fixée sur tout le tour d'une partie supérieure du corps d'allongement (3) et une deuxième extrémité de la chaussette étant fixée sur tout le tour d'un bord d'ouverture du logement (2).

15. Dispositif orthopédique (1) selon l'une quelconque des revendications précédentes, **caractérisé en ce que** l'au moins un élément limiteur de passage (6) est un corps de torsion qui peut tourner autour de l'axe longitudinal de l'élément limiteur de passage (6) d'une position initiale à une position de limitation et de la position de limitation à la position initiale, un côté étroit de l'élément limiteur de passage (6) dans une position initiale pointant dans une direction d'allongement (A) et un côté large de l'élément limiteur de passage (6) dans une position de limitation pointant dans la direction d'allongement (A).

16. Dispositif orthopédique (1) selon l'une quelconque des revendications précédentes, **caractérisé en ce que** le logement (2) comporte dans au moins une zone une membrane de compensation élastique (4) afin de compenser une variation de volume due à un changement de position du corps d'allongement (3).

17. Dispositif orthopédique (1) selon l'une quelconque des revendications précédentes, **caractérisé en ce que** la distance entre le corps d'allongement (3) et le logement (2) perpendiculairement à la direction de corps d'allongement dans la position initiale est comprise entre 0,01 et 1 000 mm, de manière préférée entre 0,1 et 50 mm, de manière particulièrement préférée entre 0,1 et 15 mm, et de manière très particulièrement préférée entre 0,1 et 5 mm.

18. Dispositif orthopédique (1) selon l'une quelconque des revendications précédentes, **caractérisé en ce que** l'au moins un élément limiteur de passage (6) comporte du caoutchouc ou du plastique, de préférence du silicone ou une matière thermoplastique, particulièrement de préférence du polypropylène, du polyéthylène et/ou du polyuréthane.

19. Dispositif orthopédique (1) selon l'une quelconque des revendications précédentes, **caractérisé en ce que** le fluide dilatant comprend une dispersion d'éthylène glycol ou d'huile de silicone et de dioxyde de silicium, de préférence du gel de silice avec une taille de particules entre 2 et 1 000 nm, une surface de 30 à 250 m²/g, une teneur de matières solides entre 5 et 80 % en poids et des stabilisateurs.
